# EUROPEAN PATENT SPECIFICATION

(11) **EP 2 357 472 B1**
(45) Date of publication and mention of the grant of the patent: **29.06.2016**
(21) Application number: 09827386.5
(22) Date of filing: 20.11.2009
(51) Int. Cl.: G01N 33/48, G01N 27/62, G01N 33/00

(54) **METHOD FOR EVALUATING STATE OF CELLS**
VERFAHREN ZUR BEURTEILUNG DES ZUSTANDES VON ZELLEN
PROCÉDÉ POUR ÉVALUER L'ÉTAT DE CELLULES

(30) Priority: 21.11.2008 JP 2008298819
(43) Date of publication of application: 17.08.2011
(73) Proprietor: National University Corporation Hokkaido University, Sapporo-shi, Hokkaido 060-0808 (JP)
(72) Inventor: NISHIMURA, Shin-Ichiro, Sapporo-shi Hokkaido 060-0808 (JP); TAKEGAWA, Yasuhiro, Sapporo-shi, Hokkaido 060-0808 (JP); AMANO, Maho, Sapporo-shi Hokkaido 060-0808 (JP)
(74) Representative: Godemeyer Blum Lenze Patentanwälte Partnerschaft mbB - werkpatent
(86) International application number: PCT/JP2009/006293
(87) International publication number: WO 2010/058605

(56) References cited:
- EP-A1- 1 362 910
- WO-A1-2008/007373
- WO-A2-2009/078015
- JP-A- 2007 278 803
- JP-A- 2008 209 261
- JAMES A. ATWOOD ET AL: "Quantitation by Isobaric Labeling: Applications to Glycomics", JOURNAL OF PROTEOME RESEARCH, vol. 7, no. 1, 1 January 2008 (2008-01-01) , pages 367-374, XP055047985, ISSN: 1535-3893, DOI: 10.1021/pr070476i
- ANNAMARI HEISKANEN ET AL: "Glycomics of bone marrow-derived mesenchymal stem cells can be used to evaluate their cellular differentiation stage", GLYCOCONJUGATE JOURNAL, KLUWER ACADEMIC PUBLISHERS, BO, vol. 26, no. 3, 27 November 2008 (2008-11-27), pages 367-384, XP019676255, ISSN: 1573-4986
- SATOMAA TERO ET AL: "The N-glycome of human embryonic stem cells", BMC CELL BIOLOGY, BIOMED CENTRAL, LONDON, GB, vol. 10, no. 1, 2 June 2009 (2009-06-02), page 42, XP021055811, ISSN: 1471-2121, DOI: 10.1186/1471-2121-10-42
- MASASHI TOYOTA ET AL.: 'Rinsho Tosa Biomarker no Kaihatsu -Tosa Kino no Kaimei to sono Oyo Dai 4 Sho Rinsho Hen 1. Tosa Kanren Biomarker no Iryo Oyo 4' SAISEI IRYO NI OKERU TOSA MARKER, IDENSHI IGAKU MOOK no. 11, 10 September 2008, pages 240 - 244
- TAKASHI MURAMATSU: 'Saibo Hyomen Shikibetsu to To Tanpakushitsu' KAGAKU TO SEIBUTSU vol. 23, no. 11, 1985, pages 709 - 716, XP008148899
- SEN'ICHIRO HAKOMORI: 'Sphingoglycolipid to Gan' BULLETIN OF THE FUJITA MEDICAL SOCIETY vol. 15, no. 2, 1991, pages 1 - 6
- NANA KAWASAKI ET AL.: 'Saibo Soshiki Riyo Iyakuhin no Hinshitsu-Anzensei To no Hyoka ni Kansuru Kiban Gijutsu Kaihatsu Kenkyu Saibo Yurai Seiri Kassei Busshitsu no Profiling Gijutsu Oyobi Kozo Kaiseki Gijutsu no Kaihatsu' KENKYU HEISEI 18 NENDO SOKATSU-BUNTAN KENKYU HOKOKUSHO 2007, pages 156 - 176 D
- JUN'ICHIRO FUJIMOTO: 'Tosa Primer-ho o Riyo shita Hakketsubyo To no Hatsugen Tosa Panel-ka to Hatsugen Tosa Probe Kaihatsu ni yoru Shindan Chiryo eno Oyo Heisei 19 Nendo Sokatsu Buntan Kenkyu Hokokusho' CHIRYO ENO OYO HEISEI 19 NENDO SOKATSU BUNTAN KENKYU HOKOKUSHO April 2008, pages 3 - 14

## Description

### [Cross-reference to related patent application]

The present application claims priority under Japanese Patent Application 2008-298819, filed on November 21, 2008.

### [Technical Field]

The present invention relates to a method for evaluating the state of cells, such as a method for evaluating the level of differentiation of stem cells or precursor cells in which differentiation has been induced. More particularly, the present invention relates to a method for evaluating the level of differentiation of stem cells or precursor cells that have been induced to differentiate into cardiomyocytes or neurons.

### [Background Art]

With the development in cellular engineering and regenerative therapy has come a need for methods of quantitatively and rapidly evaluating the state of cells. Conventionally, methods of evaluating the state of cells based on the expression levels of specific genes or proteins have been widely employed.

An example will be given below. Research on inducing differentiation in stem cells (known as, for example, iPS cells and ES cells) and precursor cells has been conducted since the 1970s. Through the 1990s, discussion of the level of differentiation of cells revolved primarily around the detection of levels of protein expression by flow cytometry, tissue staining with specific antibodies, and the like. Thus, only semi-quantitative information was obtained. However, since 2000, use of the quantitative RT-PCR method has become widespread. The absolute quantitative detection of the transcription of genes specific to differentiation has become possible (Nonpatent Reference 1). However, there are genes that are transcribed but not translated in the course of cell differentiation, and it has been suggested that the gene transcription level in and of itself does not reflect the level of differentiation (Nonpatent Reference 2).

There are also methods of absolute quantification of the level of differentiation by detecting specific proteins released into the medium after differentiation has been induced (Patent Reference 1). However, the deterioration of detection sensitivity is a concern in the detection of specific proteins. In addition, when no suitable secreted protein is present, the introduction of a reporter gene becomes necessary (Patent Reference 2).
Patent Reference 1: JP-2007-228873A
Patent Reference 2: JP-2007-37434A
Patent Reference 3: WO2004/058687

Nonpatent Reference 1: Bustin, S.A. J Mol Endocrinol. 25: 69-193 (2000)
Nonpatent Reference 2: Wen, J. et al., J Cellular Biochem 102: 149-160 (2007)
Nonpatent Reference 3: Gendai Kagaku, Tokyo Kagaku dojin, 435, 55-61(2007)
Nonpatent Reference 4: M. Yamaguchi et al., ABSTRACTS 24^{th} International
Carbohydrate Symposium. Oslo, Norway 27. July-1. August, 2008. C-P040

Patent References 1 to 3 and Nonpatent References 1 to 4 are expressly incorporated herein by reference in their entirety.

The document WO 2008/007373 A1 discloses an assay for the analysis of the glycosylation patterns, in particular for distinguishing between cell populations. The method according to WO 2008/007373 A1 specifically is directed to a method for detecting a state of a cell, comprising contacting at least a portion of the cell with at least one saccharide binding agent; determining binding of said saccharide binding agent to said cell; determining said glycosylation pattern according to binding of said at least one saccharide binding agent; and correlating said glycosylation pattern to the state of the cell. The glycosylation pattern is determined based on the binding of a binding agent to saccharides on the target cell, which are terminal sugars. The document discloses various descriptors for the state of the cells such as branching, fucose content, mannose type, sialic acid content. The document does not disclose categorizing the various sugar chains based on type of sugar chain into high mannose type and non-high mannose type and subsequently dividing the non-high mannose type into further categories.

### [Disclosure of the Invention]

### [Problem to Be Solved by the Invention]

As set forth above, tracking the state of a cell -- for example, tracking changes in the genes transcribed in the course of cell differentiation -- will generally afford good quantitative properties, but will not necessarily reflect the state of the cell (Nonpatent Reference 2).

Additionally, the detection of proteins, which are the products of translation, is a solid basis of evaluation in detecting the state of cells, such as differentiation. However, when no suitable specific antibody exists, or the detection sensitivity is poor, quantitative analysis becomes difficult, compromising general applicability.

Accordingly, the problems to be solved by the present invention are those of discovering a new indicator of the state of cells instead of genes or proteins, such as the level of differentiation; discovering a means permitting the quantitative determination, to the extent possible, of the new indicator; and providing a means of determining the level of differentiation of cells using the new indicator when differentiation is induced in specific cells.

### [Means of Solving the Problems]

The present invention takes note of the fact that changes in the state of cells, such as changes in the state of differentiation, produce quantitative changes in the sugar chain profile (Nonpatent Reference 4).

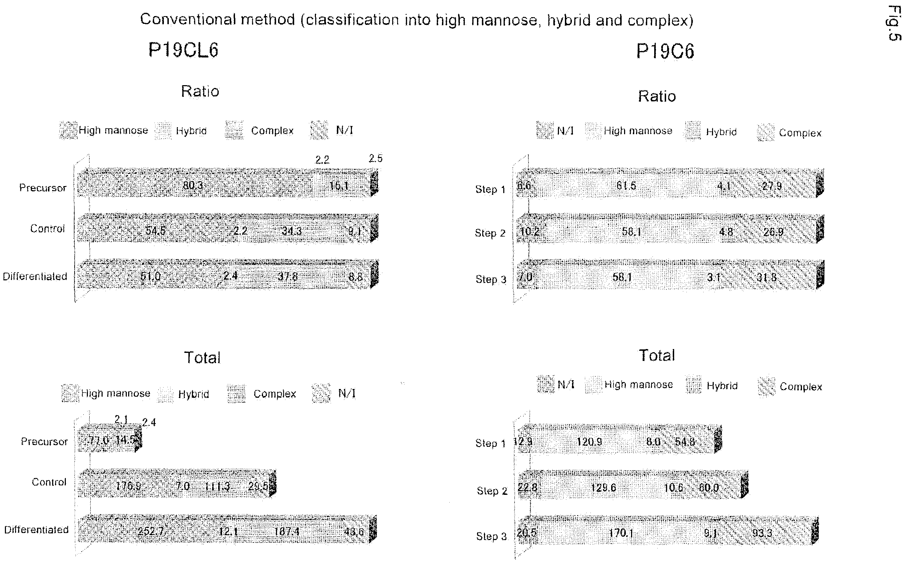

The conventional method of classifying sugar chains distinguishes the three types of high mannose, hybrid, and complex based on the biosynthesis pathway. However, the present inventors discovered that, as shown in Fig. 5, it becomes impossible to determine cell differentiation when a distinction is made between hybrid and complex types. The 'ratio' denotes a percentage (%) and the 'total' denotes an expression level (pmol). In the method of determining the sugar chain structure by mass spectrometry, there are structures for which a determination of hybrid or complex cannot be made in a single round of analysis. Such structures are denoted as N/I in the graph of Fig. 5.

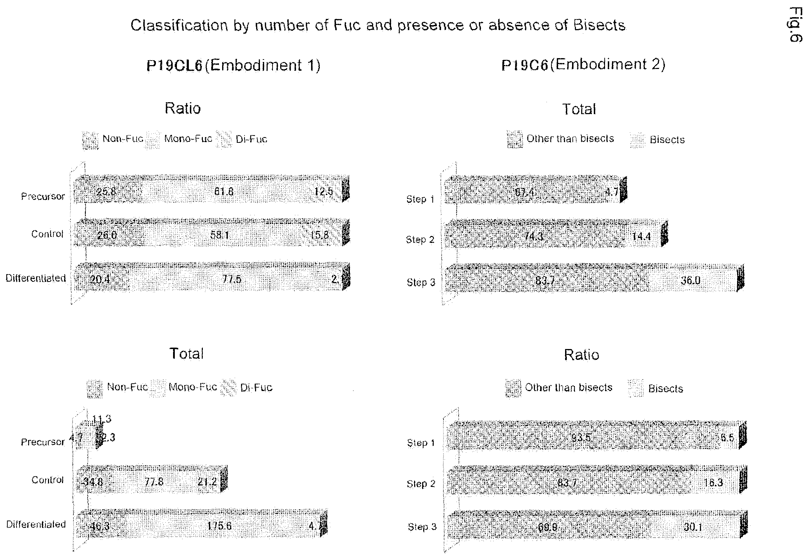

Accordingly, in the present invention, changes in the state of cells are clarified by, for example, exhaustively capturing sugar chains from cells before and after differentiation by the high-speed exhaustive sugar chain enrichment technique (glycoblotting method) (Patent Reference 3, Nonpatent Reference 3) developed by Nishimura et al., and clarifying the quantitative expression profile of sugar chains present in individual cells by mass spectrometry, such as MALDI-TOF/MS. On that basis, sugar chains were discovered that were involved in the level of cell differentiation (cell differentiation stages or types) and that varied. That is, a method of identifying sugar chains functioning as differentiation markers and varying sugar chains, and quantitatively determining the level of differentiation with high sensitivity and specificity was established. It was discovered that by introducing the concept of "sugar type" based on the information thus obtained, it became possible to divide the cells into sub-groups, and on that basis, to quantitatively determine the degree of cell differentiation. The present invention was devised on that basis (Fig. 6).

Specifically, it was discovered that cell differentiation could be quantitatively determined by the method of dividing sugar chains into the two categories of high mannose type and other (non-high mannose type), and further dividing the non-high mannose type into categories based on (1) the presence or absence of bisects, (2) the number of fucoses (0, 1, or 2 or more), (3) the presence or absence of sialic acid, and (4) the degree of branching (2 or 3 or more)

### [Effects of the Invention]

Based on the present invention, the state of cells, for example, the level of differentiation of precursor cells, stem cells, and the like -- that is, their qualitycan be quantitatively determined and managed by means of a method exceeding conventional methods in terms of sensitivity and specificity, and affording mutually complementary superiority. By permitting the quantitative measurement with high sensitivity of the level of differentiation of stem cells and the like, quality and safety management systems are enhanced and contributions to regenerative therapy can be anticipated.

### [Brief Description of Drawings]

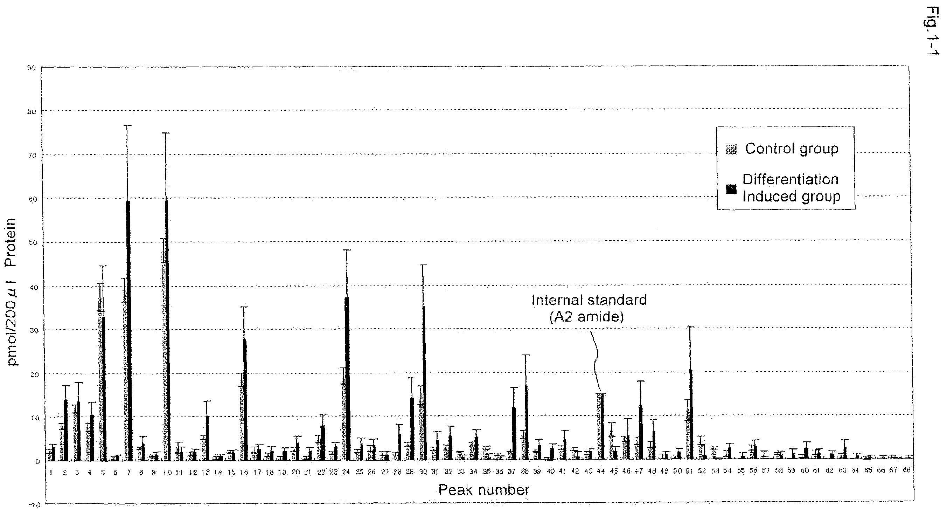

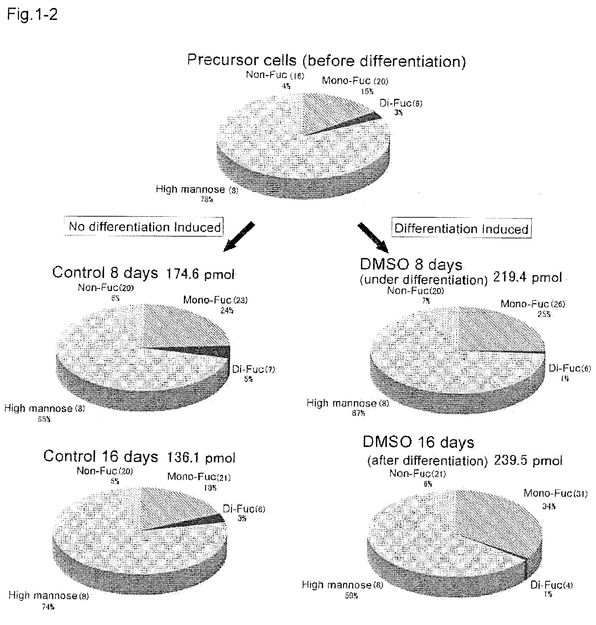

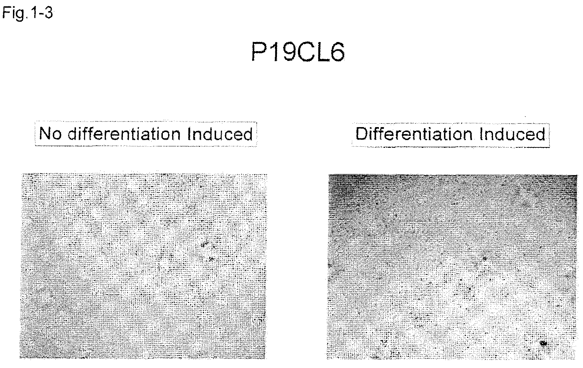

Fig. 1-1 shows a profile of the expression level of various sugar chains obtained in Embodiment 1.
Fig. 1-2 shows the results of sub-grouping by sugar chain type (mono-Fuc, di-Fuc, non-Fuc, high mannose) based on the profile obtained in Embodiment 1.
Fig. 1-3 shows the results of the immunocyte staining of cells obtained in Embodiment 1 after inducing differentiation and following culturing without inducing differentiation.
Fig. 2-1 shows the profile of expression levels of various sugar chains obtained in Embodiment 2.
Fig. 2-2 shows the results of sub-grouping by sugar chain type (bisect, non-bisect, high mannose) based on the profile obtained in Embodiment 2. The numbers in parentheses denote types of sugar chains detected.

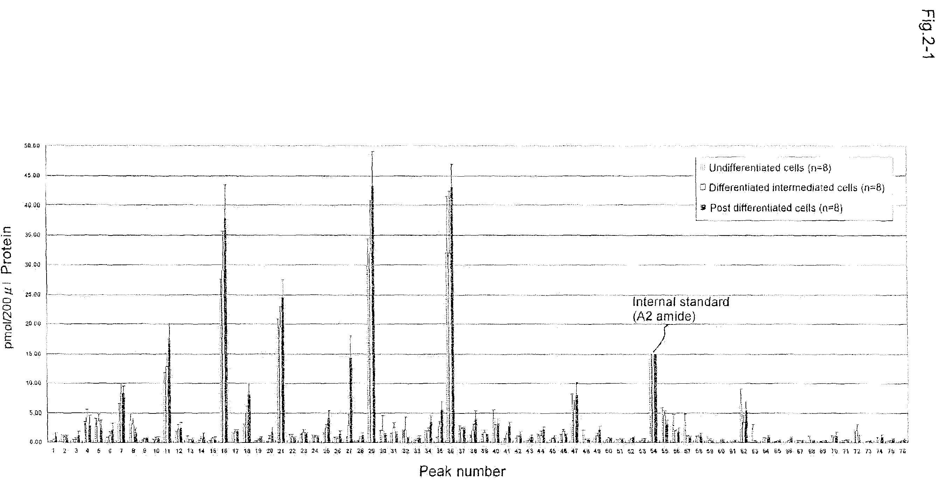

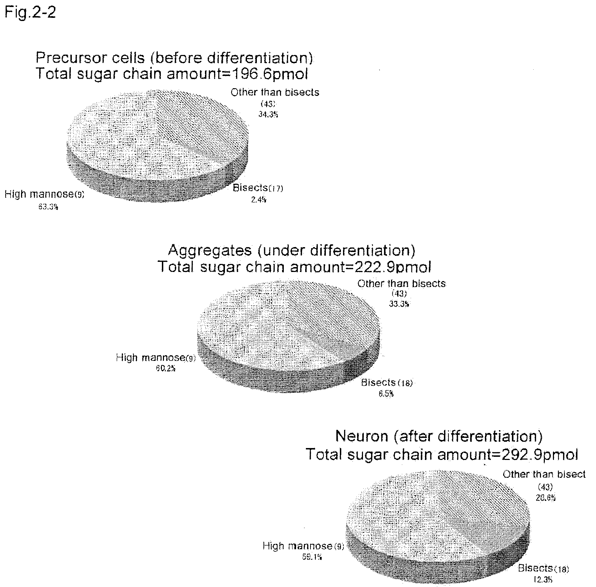

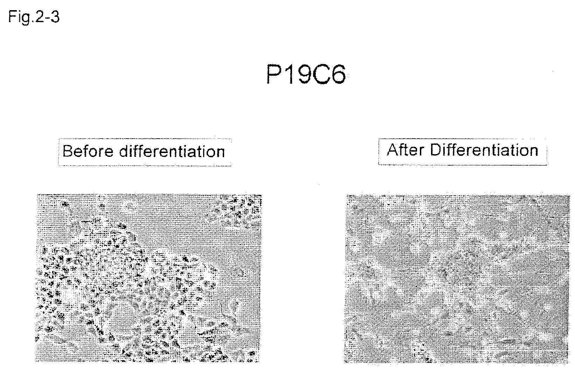

Fig. 2-3 shows the results of immunocyte staining of the cells obtained in Embodiment 2 after inducing differentiation and before differentiation.

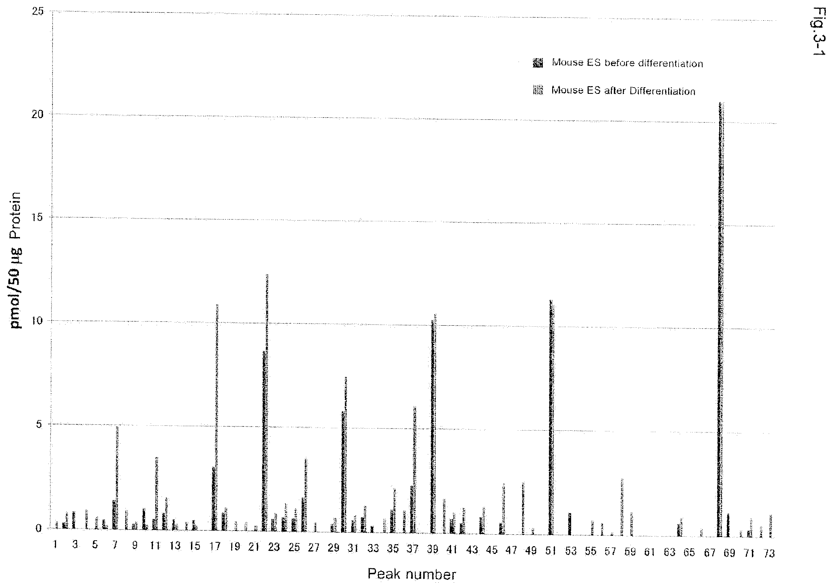

Fig. 3-1 shows the profile of expression levels of various sugar chains obtained in Embodiment 3. Peak No. 68 is an internal standard peak.

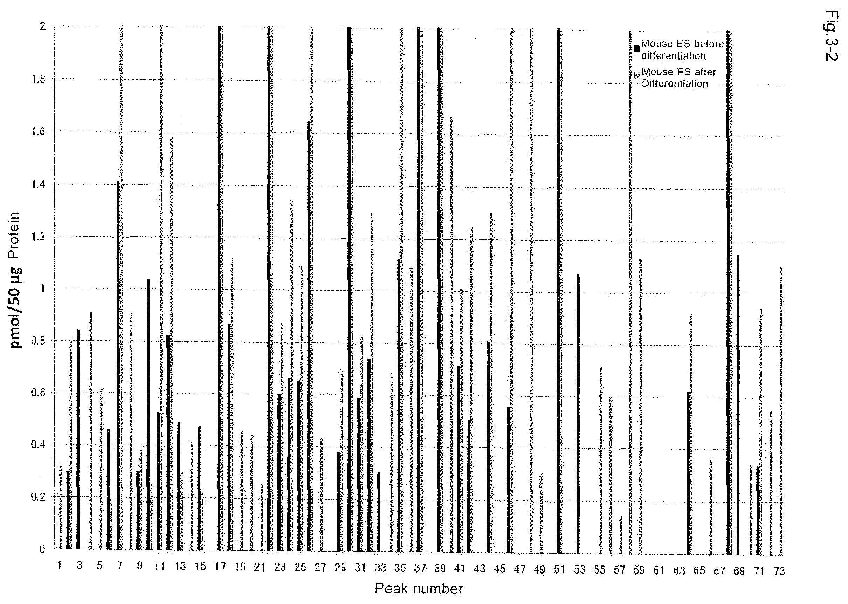

Fig. 3-2 shows the profile of expression levels of various sugar chains obtained in Embodiment 3 (an enlargement of the y-axis in Fig. 3-1).

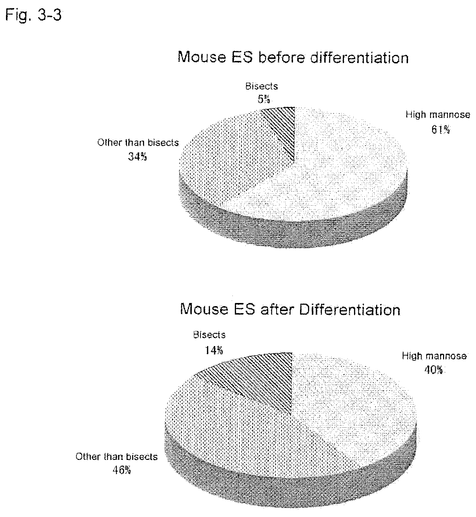

Fig. 3-3 shows the results of sub-grouping based on sugar chain type (bisect, non-bisect, high mannose) before and after differentiation based on the profile obtained in Embodiment 3.

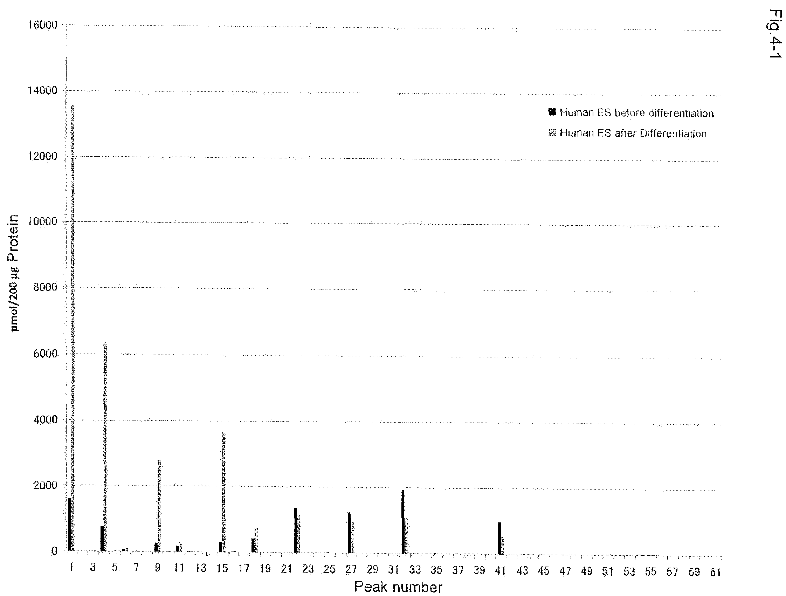

Fig. 4-1 shows the profile of expression levels of various sugar chains obtained in Embodiment 4. Peak No. 54 is an internal standard peak.

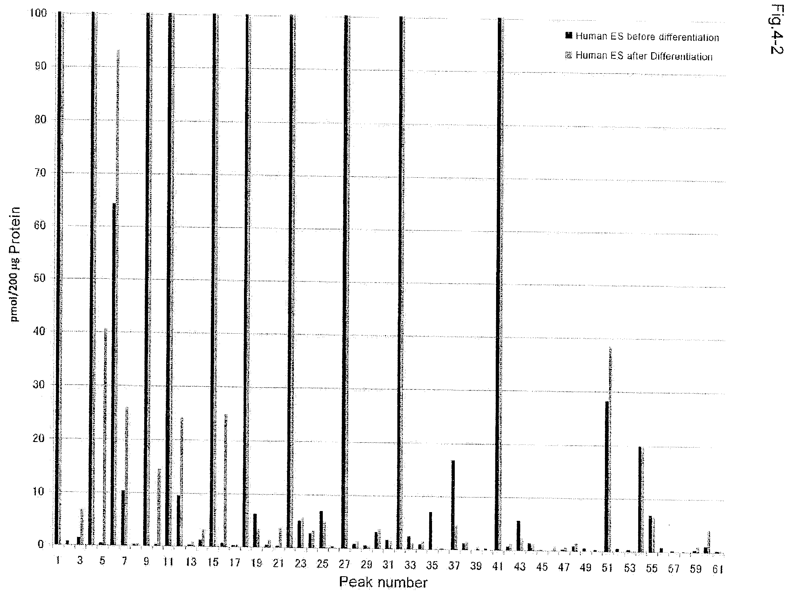

Fig. 4-2 shows the profile of expression levels of various sugar chains obtained in Embodiment 4 (an enlargement of the y-axis in Fig. 4-1).

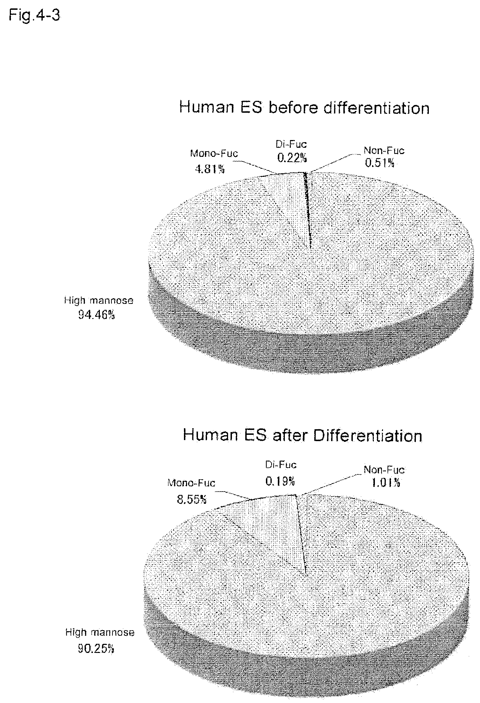

Fig. 4-3 shows the results of sub-grouping based on sugar chain type (mono-Fuc, di-Fuc, non-Fuc, high mannose) before and after differentiation based on the profile obtained in Embodiment 4.

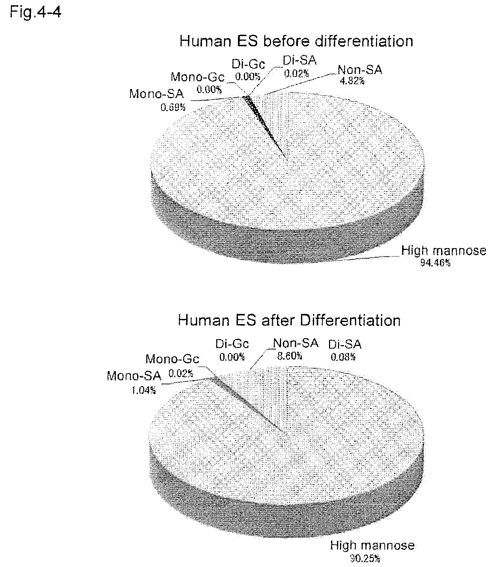

Fig. 4-4 shows the results of sub-grouping based on sugar chain type (mono-SA, di-SA, non-SA, mono-Gc, di-Gc, high mannose) before and after differentiation based on the profile obtained in Embodiment 4.
Fig. 5 shows an example of the conventional method of classifying sugar chain structures into high mannose type, hybrid-type, and complex-type. The 'ratio' denotes a percentage (%) and the 'total' denotes an expression level (pmol).
Fig. 6 shows results showing the degree of differentiation of cells obtained in Embodiments 1 and 2 by the method of the present invention. The 'ratio' denotes a percentage (%) and the 'total' denotes an expression level (pmol).

### [Modes of Carrying Out the Invention]

The present invention provides the following:
(1) A method for categorizing sugar chains employed to evaluate the level of differentiation of stem cells or precursor cells in which differentiation has been induced characterized by:
   obtaining a quantitative profile of the N-bond-type sugar chains contained in stem cells or precursor cells cultivated in the absence of a differentiation-inducing agent;
   obtaining a quantitative profile of the N-bond-type sugar chains contained in stem cells or precursor cells cultivated in the presence of a differentiation-inducing agent, wherein the N-bond-type sugar chains are those separated and purified by the glycoblotting method;
   extracting at least a part of the group of sugar chains in which the quantities present in the two quantitative profiles have varied;
   categorizing the various sugar chains contained in the group of sugar chains extracted based on type of sugar chain into high mannose type and non-high mannose type and further dividing the non-high mannose type into categories based on (1) the presence or absence of bisects, (2) the number of fucoses (0, 1, or 2 or more), (3) the presence or absence of sialic acid, and (4) the degree of branching (2 or 3 or more); and
   determining at least one category of sugar chain from among the five categories as being suited to evaluation of the level of differentiation of stem cells or precursor cells.
(2) A method for evaluating the level of differentiation of stem cells or precursor cells in which differentiation has been induced characterized by:
   obtaining time-course quantitative profiles of N-bond-type sugar chains contained in stem cells or precursor cells that have been cultured in the presence of a differentiation-inducing agent; wherein the N-bond-type sugar chains are those separated and purified by the glycoblotting method; and
   determining from the quantitative profiles the time-course change in the present quantity or present ratio of the sugar chains (groups) contained in at least one of the categories of sugar chain; wherein the at least one of the categories of sugar chain is determined by the method in which
   a quantitative profile of the N-bond-type sugar chains contained in stem cells or precursor cells cultivated in the absence of a differentiation-inducing agent is obtained;
   a quantitative profile of the N-bond-type sugar chains contained in stem cells or precursor cells cultivated in the presence of a differentiation-inducing agent is obtained, wherein the N-bond-type sugar chains are those separated and purified by the glycoblotting method;
   at least a part of the group of sugar chains in which the quantities present in the two quantitative profiles have varied is extracted;
   the various sugar chains contained in the group of sugar chains extracted based on type of sugar chain are categorized into high mannose type and non-high mannose type and the non-high mannose type is further divided into categories based on (1) the presence or absence of bisects, (2) the number of fucoses (0, 1, or 2 or more), (3) the presence or absence of sialic acid, and (4) the degree of branching (2 or 3 or more); and
   at least one category of sugar chain is determined from among the five categories as being suited to evaluation of the level of differentiation of stem cells or precursor cells.
(3) The method as described in (2), wherein the differentiation-inducing agent is a cardiac muscle differentiation-inducing agent; and
   the time-course change determined is a time-course change in at least one member selected from the group consisting of the quantity or the ratio of the group of sugar chains having one fucose, the quantity or the ratio of the group of sugar chains having two fucoses, and the quantity or the ratio of the group of sugar chains of the high mannose type present in the quantitative profile to evaluate the level of differentiation of stem cells or precursor cells into cardiac muscle.
(4) The method as described in (3), wherein cells in which the quantity or ratio of the group of sugar chains having one fucose that is present increases with culturing are cells in which the level of differentiation into cardiac muscle has increased.
(5) The method as described in (3), wherein cells in which the quantity or ratio of the group of sugar chains having two fucoses that is present decreases by culturing are cells in which the level of differentiation into cardiac muscle has increased.
(6) The method as described in (3), wherein cells in which the quantity or ratio of the group of sugar chains of the high mannose type that is present decreases with culturing are cells in which the level of differentiation into cardiac muscle has increased.
(7) The method as described in (2), wherein the differentiation-inducing agent is an agent inducing differentiation into neurons; and
   the time-course change determined is a time-course change in at least one member selected from the group consisting of the quantity or the ratio of the group of sugar chains having a bisect structure, the quantity or the ratio of the group of sugar chains not having a bisect structure, and the quantity or ratio of the group of sugar chains of the high mannose type present in the quantitative profile to evaluate the level of differentiation of stem cells or precursor cells derived from mice into neurons.
(8) The method as described in (7), wherein cells in which the quantity or ratio of the group of sugar chains having a bisect structure that is present increases with culturing are cells in which the level of differentiation into neurons has increased; wherein cells in which the quantity or ratio of the group of sugar chains not having a bisect structure that is present decreases with culturing are cells in which the level of differentiation into neurons has increased.
(9) The method as described in (7), wherein cells in which the quantity or ratio of the group of sugar chains of the high mannose type that is present decreases with culturing are cells in which the level of differentiation into neurons has increased.
(10)The method as described in (2), wherein the differentiation-inducing agent is an agent inducing differentiation into neurons; and
   the time-course change determined is a time-course change in at least one member selected from the group consisting of the quantity or the ratio of the group of sugar chains having one fucose, the quantity or the ratio of the group of sugar chains not having sialic acid, and the quantity or ratio of the group of sugar chains of the high mannose type present in the quantitative profile to evaluate the level of differentiation of stem cells or precursor cells derived from humans into neurons.
(11) The method as described in (10), wherein cells in which the quantity or ratio of the group of sugar chains having one fucose that is present increases with culturing are cells in which the level of differentiation into neurons has increased; wherein cells in which the quantity or ratio of the group of sugar chains not having sialic acid that is present increases with culturing are cells in which the level of differentiation into neurons has increased.
(12) The method as described in (10), wherein cells in which the quantity or ratio of the group of sugar chains of the high mannose type that is present decreases with culturing are cells in which the level of differentiation into neurons has increased.
(13) The method as described in any one of (1) to (12), wherein the quantitative profiles are those obtained through the use of MALDI-TOF/MS or LC-ESI/SSI-TOF/MS.
(14) The method as described in any one of (1) to (13), wherein the stem cell is embryonic stem cells, tissue stem cells, adult stem cells, or induced pluripotent stem cells (iPS cells).
(15) The method as described in any one of claims (1) to (14), wherein the precursor cell is a cell obtained by inducing differentiation in a stem cell and is pluripotent.

[The method for categorizing sugar chains used to evaluate the differentiation level of stem cells or precursor cells]

The first aspect of the present invention is a method for categorizing sugar chains employed to evaluate the level of differentiation of stem cells or precursor cells in which differentiation has been induced. This method is comprised of the following steps:
(3-1) obtaining a quantitative profile of the N-bond-type sugar chains contained in stem cells or precursor cells cultivated in the absence of a differentiation-inducing agent;
(3-2) obtaining a quantitative profile of the N-bond-type sugar chains contained in stem cells or precursor cells cultivated in the presence of a differentiation-inducing agent;
(3-3) extracting at least a part of the group of sugar chains in which the quantities present in the two quantitative profiles have varied;
(3-4) categorizing the various sugar chains contained in the group of sugar chains extracted based on type of sugar chain into high mannose type and non-high mannose type and further dividing the non-high mannose type into categories based on (1) the presence or absence of bisects, (2) the number of fucoses (0, 1, or 2 or more), (3) the presence or absence of sialic acid, and (4) the degree of branching (2 or 3 or more); and
(3-5) determining at least one category of sugar chain from among the five categories as being suited to evaluation of the level of differentiation of stem cells or precursor cells.

### Steps (3-1) and (3-2)

In step (3-1), a quantitative profile of the N-bond-type sugar chains contained in stem cells or precursor cells cultivated in the absence of a differentiation-inducing agent is obtained. In step (3-2), a quantitative profile of the N-bond-type sugar chains contained in stem cells or precursor cells cultivated in the presence of a differentiation-inducing agent is obtained. Steps (3-1) and (3-2) can be successively conducted, or simultaneously conducted in parallel. When conducted successively, either step can be conducted first.

In the present invention, including additional aspects thereof set forth below, the term "stem cell" means a cell having multipotency or pluripotency. Examples of "stem cells" are embryonic stem cells, tissue stem cells, adult stem cells, and induced pluripotent stem cells (iPS cells). However, any cell that is multipotent or pluripotent is included as a stem cell, without limitation to the above examples.

In the present invention, including additional aspects thereof set forth below, the term "precursor cell" means a cell that is obtained by inducing differentiation in the above stem cell and that is pluripotent. When differentiation is induced in a stem cell, depending on the degree of differentiation, there are cells that have a lower degree of undifferentiation than stem cells while still being pluripotent. In the present invention, such cells are collectively referred to as "precursor cells." "Precursor cells" are not specifically limited other than that they be pluripotent. The P19CL6 and P19C6 cell strains employed in Embodiments 1 and 2 are both precursor cells.

The above "stem cells" and "precursor cells" can be derived from animals such as mammals and fish. Examples of mammals are: humans, mice, rats, sheep, pigs, and monkeys. Examples of fish are: killifish and zebrafish. However, they are not limited to these examples.

In the first aspect of the present invention, a known method of culturing stem cells or precursor cells can be used as the method of culturing stem cells or precursor cells in the presence or absence of a differentiation-inducing agent. The differentiation-inducing agent is not specifically limited, and can be suitably selected based on the type of stem cell or precursor cell. However, since the present invention is a method for categorizing sugar chains for use in evaluating the level of differentiation of stem cells or precursor cells, the use of a differentiation-inducing agent that is capable of highly efficiently and reliably inducing differentiation is desirable from the perspective facilitating sugar chain categorization. However, there are cases where the differentiation level (the differential stage or type of the cell) varies with the type of differentiation-inducing agent. Thus, the use of a differentiation-inducing agent that is capable of reliably inducing differentiation is not always called for; different differentiation-inducing agents can be used based on the differentiation level being targeted. A single differentiation-inducing agent or a combination of such agents can be employed in culturing. The concentration of the differentiation-inducing agent in the culture (medium) can also be suitably selected.

The cultured stem cells or precursor cells are subjected to processing to separate and purify the N-bond-type sugar chains contained in the cells. Quantitative profiles of the separated and purified N-bond-type sugar chains are suitably obtained. By way of example, the N-bond-type sugar chains are desirably separated and purified by the glycoblotting method described in Patent Reference 3 and Nonpatent Reference 3 from the perspectives of rapid separation and purification without loss of N-bond-type sugar chains. Quantitative profiling is suitably conducted to a certain degree or higher of differentiation of stem cells or precursor cells that have been cultured with a differentiation-inducing agent, that is, to a degree where the progress of differentiation is evident. However, the degree at which the progress of differentiation becomes evident varies with the type of cell, type of differentiation-inducing agent, culturing conditions, and the like. Thus, a suitable determination is made taking these conditions into account.

The quantitative profiles are desirably obtained for separated, purified N-bond-type sugar chains by matrix-assisted laser desorption/ionization-time of flight/mass spectrometry (MALDI-TOF/MS) or liquid chromatography-electrospray ionization/sonic spray ionization-time of flight/mass spectrometry (LC-ESI/SSI-TOF/MS). N-bond-type sugar chains can be rapidly quantified with high precision and quantitative profiles can be obtained through the use of MALDI-TOF/MS or LC-ESI/SSI-TOF/MS.

### Steps (3-3) and (3-4)

In step (3-3), at least a part of the group of sugar chains in which the quantities present in the two quantitative profiles obtained in steps (3-1) and (3-2) have varied is extracted. In step (3-4), the various sugar chains contained in the group of sugar chains extracted based on type of sugar chain are categorized into high mannose type and non-high mannose type and the non-high mannose type is further divided into categories based on (1) the presence or absence of bisects, (2) the number of fucoses (0, 1, or 2 or more), (3) the presence or absence of sialic acid, and (4) the degree of branching (2 or 3 or more).

As a result of experimentation, the present inventors discovered that these five categories of sugar chains could be used to evaluate the differentiation levels of stem cells and precursor cells.

There are cases in which the group of sugar chains in the quantitative profiles differs with the type of cell and the direction and degree of differentiation. However, it is normally comprised of about several tens to several hundred sugar chains. Of these, at least a part of the group of sugar chains in which the quantity present varies based on the differentiation level is extracted, and the group of sugar chains that is extracted is divided into five categories based on the above types of sugar chain.

### Step (3-5)

In step (3-5), at least one category of sugar chain from among the five categories is determined to be suited to evaluation of the state of the cells. For example, the category of sugar chain that is suited to the evaluation of the state of the cells can be one such that time-course variation in the quantity present or the ratio present is not significant in stem cells or precursor cells that have been cultured in the absence of a differentiation-inducing agent, but time-course variation in the quantity present or the ratio present is significant in stem cells or precursor cells that have been cultured in the presence of a differentiation-inducing agent.

The category of sugar chain that is selected and determined here can be used as an evaluation index of the level of differentiation in the method for evaluating the level of differentiation of stem cells or precursor cells in which differentiation has been induced as set forth below.

[The method for evaluating the level of differentiation of stem cells or precursor cells in which differentiation has been induced]

The second aspect of the present invention is a method for evaluating the level of differentiation of stem cells or precursor cells in which differentiation has been induced. This method comprises the following steps:
(4-1) obtaining time-course quantitative profiles of N-bond-type sugar chains contained in stem cells or precursor cells that have been cultured in the presence of a differentiation-inducing agent; and
(4-2) determining from the quantitative profiles the time-course change in the present quantity or present ratio of the sugar chains (groups) contained in at least one of the categories of sugar chain determined by the method of the first aspect of the present invention.

### Step (4-1)

In step (4-1), the time-course quantitative profiles of N-bond-type sugar chains contained in stem cells or precursor cells that have been cultured in the presence of a differentiation-inducing agent can be obtained in the same manner as in step (3-2) above. However, the time-course quantitative profiles is determined in step (4-1). Specifically, the cultured product is sampled over the course of the culturing period, and quantitative profiles are obtained for N-bond-type sugar chains that have been separated and purified from the sampled cultured product. The time-course quantitative profiles that are obtained are obtained at least once, desirably two or more times, and for example, two, three, or four times, following the start of culturing. They can also be obtained more than four times.

### Step (4-2)

In step (4-2), the time-course change in the quantity or ratio of sugar chains (groups) present in the quantitative profiles is determined for at least one of the categories of sugar chain determined by the method of the first aspect of the present invention.

A sugar chain type (sugar chain category) that can be employed as a differentiation marker in the method of evaluating the level of neuron differentiation and in the method of evaluating the level of cardiac muscle differentiation described further below was discovered by means of the sugar chain category-determining method of the first aspect of the present invention. The details are given in the embodiments. However, it was discovered that at least one member selected from the group consisting of the group of sugar chains having one fucose and the group of sugar chains having two fucoses could be employed as a differentiation marker in the method of evaluating the level of differentiation of cardiac muscle. Further, it was discovered that at least one member selected from the group of sugar chains having a bisect structure and the group of sugar chains not having a bisect structure could be employed as a differentiation marker in the method of evaluating the level of differentiation of neurons.

The method for evaluating the level of differentiation using the above differentiation markers will be described below.

### [The method for evaluating the level of differentiation of cardiac muscle]

A preferred embodiment of the second aspect of the present invention is a method for evaluating the level of differentiation of stem cells or precursor cells into cardiac muscle.

This method comprises:
(5-1) obtaining a time-course quantitative profile of N-bond-type sugar chains contained in stem cells or precursor cells cultured in the presence of a cardiac muscle differentiation-inducing agent; and
(5-2) determining a time-course change in at least one member selected from the group consisting of the quantity or the ratio of the group of sugar chains having one fucose, the quantity or the ratio of the group of sugar chains having two fucoses, and the quantity or the ratio of the group of sugar chains of the high mannose type present in the quantitative profile.

### Step (5-1)

Examples of cardiac muscle differentiation-inducing agents include dimethylsulfoxide (DMSO) and oxytocin. The time-course quantitative profile of N-bond-type sugar chains can be obtained in the same manner as in step (3-2) in the first aspect set forth above. However, the differentiation-inducing agent employed is a cardiac muscle differentiation-inducing agent.

The stem cells or precursor cells employed in the method of the present invention are the same as those employed in the first aspect. In particular, P19CL6 cells, ES cells, iPS cells, and the like are preferred.

### Step (5-2)

At least one member selected from the group consisting of the quantity or the ratio of the group of sugar chains having one fucose, the quantity or the ratio of the group of sugar chains having two fucoses, and the quantity or the ratio of the group of sugar chains of the high mannose type present in the quantitative profile is determined. In the present invention, the level of differentiation of cardiac muscle can be evaluated based on the quantity or ratio of any of the group of sugar chains present. However, it is desirably collectively evaluated based on the quantities or ratios of all three of these groups of sugar chains that are present.

An example of a group of sugar chains having one fucose is Chem. 3, Chem. 19, and Chem. 44 in Table 1.

An example of a group of sugar chains having two fucoses is Chem. 20, Chem. 41, and Chem. 49 in Table 1.

An example of a group of sugar chains of the high mannose type is Chem. 5, Chem. 8, and Chem. 14 in Table 1.

As indicated in Embodiment 1, cells presenting a quantitative profile in which the quantity or ratio of the group of sugar chains having one fucose that is present increases with culturing are cells that have undergone an increase in the level of differentiation of cardiac muscle. Similarly, cells presenting a quantitative profile in which the quantity or ratio of the group of sugar chains having two fucoses is decreased by culturing are cells that have undergone an increase in the level of differentiation of cardiac muscle.

### [The method for evaluating the level of differentiation of neurons (1)]

A preferred embodiment of the second aspect of the present invention is a method for evaluating the level of differentiation of stem cells or precursor cells derived from mice into neurons.

This method comprises:
(6-1) obtaining a time-course quantitative profile of N-bond-type sugar chains contained in stem cells or precursor cells cultured in the presence of an agent inducing differentiation into neurons; and
(6-2) determining a time-course change in at least one member selected from the group consisting of the quantity or the ratio of the group of sugar chains having a bisect structure, the quantity or the ratio of the group of sugar chains not having a bisect structure, and the quantity or ratio of the group of sugar chains of the high mannose type present in the quantitative profile.

### Step (6-1)

Examples of agents inducing differentiation into neurons include retinoic acid and DMSO. The time-course quantitative profile of N-bond-type sugar chains can be obtained in the same manner as in step (3-2) of the first aspect. However, the differentiation-inducing agent employed is an agent inducing differentiation into neurons.

The stem cells or precursor cells employed in the method of the present invention are the same as those employed in the first aspect. In particular, P19 cells, P19C6 cells, ES cells, iPS cells, and the like are preferred.

### Step (6-2)

At least one member selected from the group consisting of the quantity or the ratio of the group of sugar chains having a bisect structure, the quantity or the ratio of the group of sugar chains not having a bisect structure, and the quantity or ratio of the group of sugar chains of the high mannose type present in the quantitative profile is determined. In the present invention, the level of differentiation into neurons can be evaluated based on the quantity or ratio of any one group of sugar chains present, but is desirably collectively evaluated based on the quantities or ratios of all three groups of sugar chains present.

The term "bisect structure" refers to the structure of a sugar chain having a bisect GlcNAc. When a bisect GlcNAc is added to the structure of the skeletal sugar chain, the extension of further branch divisions and the severing of sugar chains are known to cease. The reaction that adds a bisect GlcNAc to the sugar chain structure is catalyzed by an enzyme known as GnT-III (Ikeda, Y., et al., Trends in Glycoscience and Glycotechnology 13: 167-176.

An example of a group of sugar chains having a bisect structure is: Chem. 15, Chem. 45, and Chem. 68 in Table 2.

An example of a group of sugar chains not having a bisect structure is: Chem. 17, Chem. 21, and Chem. 71 in Table 2.

An example of a group of sugar chains of the high mannose type is the same as that given by way of example for the method steps (5-1) and (5-2) described above.

As indicated in Embodiment 2, in mouse precursor cells, cells in which the quantity or ratio of the group of sugar chains having a bisect structure that is present increases with culturing are cells in which the level of differentiation into neurons has increased. Cells in which the quantity or ratio of the group of sugar chains not having a bisect structure that is present decreases with culturing are cells in which the level of differentiation into neurons has increased. And cells in which the quantity or ratio of the group of sugar chains of the high mannose type that is present decreases with culturing are cells in which the level of differentiation into neurons has increased.

As indicated in Embodiment 3, in mouse ES cells, cells in which the quantity or ratio of the group of sugar chains having a bisect structure that is present increases with culturing are cells in which the level of differentiation into neurons has increased. Cells in which the quantity or ratio of the group of sugar chains not having a bisect structure that is present decreases with culturing are cells in which the level of differentiation into neurons has increased. And cells in which the quantity or ratio of the group of sugar chains of the high mannose type that is present decreases with culturing are cells in which the level of differentiation into neurons has increased.

### [The method for evaluating the level of differentiation into neurons (2)]

A preferred embodiment of the second aspect of the present invention is a method for evaluating the level of differentiation of stem cells or precursor cells derived from humans into neurons.

This method comprises:
(7-1) obtaining a time-course quantitative profile of N-bond-type sugar chains contained in stem cells or precursor cells cultured in the presence of an agent inducing differentiation into neurons; and
(7-2) determining a time-course change in at least one member selected from the group consisting of the quantity or the ratio of the group of sugar chains having one fucose, the quantity or the ratio of the group of sugar chains not having sialic acid, and the quantity or ratio of the group of sugar chains of the high mannose type present in the quantitative profile.

(7-1) As in step (6-1), examples of agents inducing differentiation into neurons include retinoic acid and DMSO. A time-course quantitative profile of N-bond-type sugar chains can be obtained in the same manner as in step (3-2) of the first aspect. However, the differentiation-inducing agent employed is an agent inducing differentiation into neurons.

The human-derived stem cells or precursor cells employed in the method of the present invention are the same as those indicated in the first aspect above. In particular, ES cells, iPS cells, and the like are preferred.

### Step (7-2)

Time-course change in at least one member selected from the group consisting of the quantity or the ratio of the group of sugar chains having one fucose, the quantity or the ratio of the group of sugar chains not having sialic acid, and the quantity or ratio of the group of sugar chains of the high mannose type present in the quantitative profile is determined. In the present invention, the level of differentiation into neurons can be evaluated based on the quantity or ratio of any one group of sugar chains present, but is desirably collectively evaluated based on the quantities or ratios of all three groups of sugar chains present.

Examples of sugar chains having one fucose, sugar chains not having sialic acid, and sugar chains of the high mannose type are the sugar chains indicated in Table 4 of Embodiment 4.

As indicated in Embodiment 4, in human ES cells, cells in which the quantity or ratio of the group of sugar chains having one fucose that is present increases with culturing are cells in which the level of differentiation into neurons has increased. Cells in which the quantity or ratio of the group of sugar chains not having sialic acid (Non-SA) that is present increases with culturing are cells in which the level of differentiation into neurons has increased. And cells in which the quantity or ratio of the group of sugar chains of the high mannose type that is present decreases with culturing are cells in which the level of differentiation into neurons has increased.

In the preferred embodiments attention is paid in the embodiments to the tendency of the quantity or ratio of the various groups of sugar chains present to time-course change. However, by obtaining and gathering information relating to the relation between the stage or type of differentiation and the time-course change of the quantities or ratios of the various groups of sugar chains that are present, it is possible to quantitatively determine the stage and type of differentiation from the quantity or ratio of the various groups of sugar chains that are present. For example, when employing differentiating cells in regenerative therapy, it is desirable in terms of safety not to incorporate cells that are undifferentiated. Accordingly, it is important to correctly determine the stage of differentiation of the cells obtained by culturing. It is also important to confirm that the cells are differentiated into the targeted type, and thus, to correctly determine the type of differentiation. The method of the present invention permits quantitative determination of the stage and type of differentiation from the quantities or ratios of the groups of sugar chains that are present.

### [Embodiments]

The present invention will be described in greater detail below through embodiments.

### Cell glycomics protocols

The cell glycomics employed in the embodiments were implemented according to the following protocols.

### <Preparation of cell lysate and release of N-glycan>

1. In 10 mM EDTA/PBS, a cell scraper is used to recover cells from a dish. The cells are washed with PBS.
2. On percent (final concentration) of TritonX-100 is added, the cells are incubated for one hour on ice, and the cells are solubilized.
3. Eighty percent (final concentration) of acetone is added and incubation is conducted over night at -20°C to cause the protein fraction to precipitate.
4. Following centrifugation, the precipitate is washed with acetonitrile and the acetone is completely removed.
5. The precipitate is blown dry, 50 µL of 80 mM ammonium bicarbonate containing 0.2% PHM (1-propanesulfonic acid, 2-hydroxyl-3-myristamido) is added, and the mixture is incubated at 60°C for 10 minutes and solubilized.
6. DTT is added to a final concentration of 10 mM to the protein fraction following solubilization, and the mixture is reduced at 60°C for 30 minutes.
7. To this is added 20 mM iodoacetamide and the mixture is incubated for 30 minutes at room temperature while being shielded from light to alkylate the amino acid residue reduced in 6.
8. 800 U of trypsin is added and the mixture is incubated over night at 37°C.
9. The trypsin is activated for 10 minutes at 90°C, 2 U of peptide N-glycanase F is added, and N-bond-type sugar chains were released.
10. An internal standard in the form of 15 pmol of A2 amide glycan is added to the sugar chains that had been released, after which the mixture is concentrated in a SpeedVac and adjusted to a final volume of 20 µL.

### <Glycoblotting>

The N-bond-type sugar chains obtained in 10. above were enriched by the glycoblotting method using a BlotGlyco^{(R)} H (Sumitomo Bakelite Co., Ltd.). This method was implemented in accordance with the methods described in the following references. The specific method is described in the references cited below.
- Furukawa J-i., et al., Anal. Chem., 4: 1094-1101 (2008)
- Baudino L., et al., J. Immunol., 181: 4107-4112 (2008)
- Baudino L., et al., J. Immunol., 181: 6664-6669 (2008)
- Uematsu R., et al., Mol. Cell. Proteom., in press (2008)

### <Glycoblotting using BlotGlyco^{(R)} H beads>

1. N-bond-type sugar chains, which have been released with peptide N-glycanase F, to which 15 pmol of A2 amide glycan has been added as an internal standard, which has been concentrated with a SpeedVac, and the final volume of which has been adjusted to 20 µL, are added to 5 mg of BlotGlyco^{(R)} H beads.
2. A 180 µL quantity of acetonitrile containing 2% of acetic acid is added to 1.
3. The mixture is left standing at 80°C for 45 minutes.
4. The beads are washed twice with 200 µL each of 16.6 mM ammonium bicarbonate solution containing 2 M guanidine-hydrochloric acid, pure water, and a 1% triethylamine methanol solution.
5. A 100 µL quantity of a methanol solution containing 10% acetic anhydride is added and the mixture is left standing at room temperature for 30 minutes.
6. After removing the solution, the beads are washed twice with 200 µL each of 10 mM hydrochloric acid, methanol, and dioxane.
7. A 100 µL quantity of dioxane solution containing 100 mM 3-methyl 1-p-tolyltriazene is added and the mixture is left standing at 60°C for an hour to an hour and a half.
8. The mixture is sequentially washed with 200 µL each of dioxane, pure water, methanol, and pure water.
9. A 180 µL quantity of acetonitrile containing 2% acetic acid and 20 µL of 20 mM aminooxy-WR is added.
10. The mixture is left standing at 80°C for 45 minutes.
11. The mixture is efuted from 100 µL of pure water.

### Embodiment 1

Differentiation into cardiomyocytes was induced in P19CL6 cells derived from mouse embryonal carcinoma cells, and glycomixes of undifferentiated and differentiated cells were prepared.

Mouse embryonal carcinoma cell-derived P19Cl6 cells were inoculated onto DMEM medium containing 10% bovine fetal serum to 3.7 x 10⁵ cells/6 cm dish. To the group in which differentiation was being induced was added 1% dimethylsulfoxide (DMSO) and culturing was conducted. The control was cultured as is, without addition. On day 16 following the start of culturing, pulsation of the entire differentiation-induced group was confirmed and cells were collected from both groups. The above glycoblotting method was used to capture and purify all the N-bond-type sugar chains in the cells. A quantity of total cell protein corresponding to 200 µg was analyzed, and a quantitative profile was obtained by MALDI-TOF/MS. The expression quantities of the various sugar chains shown in Fig. 1-1 are given as absolute quantitative values of sugar chains calculated from the area ratios in a spectrum to which an oligosaccharide of known quantity had been added as an internal standard. Based on these results, 42 sugar chains exhibiting significant variation were confirmed. A tendency to increase with differentiation was observed in 11 types of sugar chain having one Fuc, and a significant reduction was observed in the differentiation-induced group in six structures of sugar chains having two Fucs. Sub-grouping by sugar chain type in this manner was determined to permit quantitative determination of the level of cell differentiation (Fig. 1-2).

Immunocyte staining was conducted under the following conditions on cells in which differentiation had been induced and on cells that had been cultured without inducing differentiation. The results are given in Fig. 1-3. The expression of sarcomere myosin, a protein specific to cardiomyocytes, was confirmed in just those cells in which differentiation had been induced. It was not discovered in cells that had been cultured without inducing differentiation. Thus, only those cells in which differentiation had been inducted had differentiated into cardiomyocytes.
Primary antibody: MF20 (mouse monoclonal anti-sarcomeric myosin)
Secondary antibody: HRP-polymer conjugated anti-IgG
Color-developing substrate: Diaminobenzidine (DAB)

Pulsation was confirmed (by visual evaluation) in cells in which differentiation had been induced and in cells that had been cultured without inducing differentiation. As a result, pulsation was not observed in the group in which differentiation had not been induced, but was observed in about 80% of the cells in the group in which differentiation had been induced.

Of the 42 sugar chains in which significant variation was observed, the 11 sugar chains having one Fuc, the six sugar chains having two Fucs, and the sugar chains of the high mannose type are indicated below.

The group of sugar chains having one fucose is: Chem. 2, Chem. 3, Chem. 6, Chem. 10, Chem. 12, Chem. 15, Chem. 16, Chem. 19, Chem. 24, Chem. 25, Chem. 27, Chem. 30, Chem. 31, Chem. 33, Chem. 36, Chem. 37, Chem. 39, Chem. 40, Chem. 41, and Chem. 42 in Table 1.

The group of sugar chains having two fucoses is: Chem. 20, Chem. 22, Chem. 28, Chem. 29, Chem. 34, Chem. 35, and Chem. 38 in Table 1.

The sugar chains of the high mannose type are: Chem. 5, Chem, 8, Chem. 14, and Chem. 17.

**[Table 1]**

| Structure name | Assumed structure | Structure name | Assumed structure |
|---|---|---|---|
| Chem.1 | 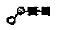 | Chem.22 | 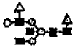 |
| Chem.2 | 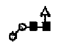 | Chem.23 | 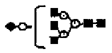 |
| Chem.3 | 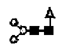 | Chem.24 | 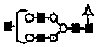 |
| Chem.4 | 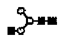 | Chem.25 | 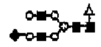 |
| Chem.5 | 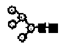 | Chem.26 | 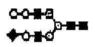 |
| Chem.6 | 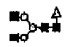 | Chem.27 | 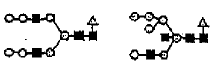 |
| Chem.7 | 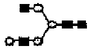 | Chem.28 | 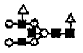 |
| Chem.8 | 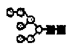 | Chem.29 | 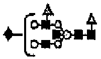 |
| Chem.9 | 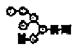 | Chem.30 | 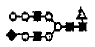 |
| Chem.10 | 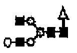 | Chem.31 | 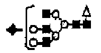 |
| Chem.11 | 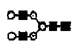 | Chem.32 | 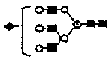 |
| Chem.12 | 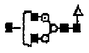 | Chem.33 | 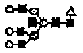 |
| Chem.13 | 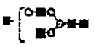 | Chem.34 | 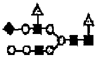 |
| Chem.14 | 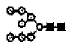 | Chem.35 | 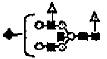 |
| Chem.15 | 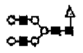 | Chem.36 | 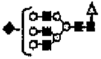 |
| Chem.16 | 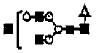 | Chem.37 | 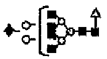 |
| Chem.17 | 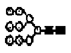 | Chem.38 | 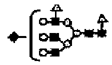 |
| Chem.18 | 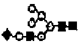 | Chem.39 | 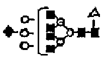 |
| Chem.19 | 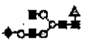 | Chem.40 | 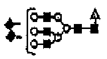 |
| Chem.20 | 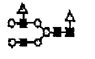 | Chem.41 | 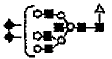 |
| Chem.21 | 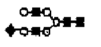 | Chem.42 | 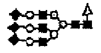 |

In table, ■ donates N-acetyl glucosamine (GlcNAc), Δdonates Fucose (Fuc), ○ (grayish) donates Mannose (Man), Odonates Galactose (Gal), ● donates Glucose (Glc), ◊ donates N-acetyl sialic acid (Neu Ac) and ◊(grayish) donates N-glycolyl sialic acid (Neu Gc).

### Embodiment 2

Mouse embryonic carcinoma P19C6 cells were induced to differentiate into neurons and glycomixes were prepared for three steps of undifferentiated, intermediate differentiation, and differentiated cells.

### Step 1: Undifferentiated cells

P19C6 cells were inoculated and cultured for several days in a 15% FBS/DMEM medium in a cell culture dish. When 80 to 90% confluence was reached, the cells were recovered and employed to prepare a glycomix or used in step 2 for the induction of differentiation.

### Step 2: Intermediate differentiation cells

Cells that had reached 80 to 90% confluence in step 1 were processed with trypsin to prepare a cell suspension and hanging drop cultured in a bacteria grade Petri dish. At the time, 100 spots of 10 µL cell suspension (10⁵ cells/mL) were applied to the cover of a 10 cm dish and float-culturing was conducted to form lumps of cell aggregation. Ten percent FBS/aMEM to which 1 µM retinoic acid had been added to induce differentiation was employed as the medium. The cell aggregates were collected after four days. They were employed to prepare a glycomix and in step 3 to induce differentiation.

### Step 3: Differentiated cells

The recovered lumps of cell aggregate were processed with trypsin to prepare a cell suspension. The cells were inoculated to 5 x 10⁴ cells/cm² on a 6 cm dish coated with polylysine and adhesion cultured. DMEM/F12 to which N-2 supplement and fibronectin had been added was employed as the medium. The cells were recovered after five days and used to prepare a glycomix.

### The glycomix

Proteins in the pellets of the recovered cells were solubilized with 1 % Triton/PBS. The equivalent of 200 µg of protein was precipitated from acetone, reduced/alkylated, and digested with trypsin. Subsequently, the N-bond-type sugar chains were released with PNGaseF, glycoblotting was used to capture and purify the sugar chains, and a quantitative profile was obtained by MALDI-TOF/MS. The expression levels of the sugar chains were quantitatively analyzed by comparing the areas of the signals derived from the sugar chains expressed by the cells to the area of the signal derived from an oligosaccharide of known concentration that was added as an internal standard in the spectra of the undifferentiated, intermediate differentiation, and differentiated cells (Fig. 2-1). As a result, a significant change in sugar chain structure was found to accompany differentiation. Based on this result, sub-grouping into structures thought to having bisect GlcNAc and sugar chains thought to lack it clearly permitted the quantitative monitoring of cell differentiation, as shown in Fig. 2-2.

Immunocyte staining was conducted under the following conditions on cells in which differentiation had been induced and on P19C6 cells in an undifferentiated state. The results, as given in Fig. 2-3, reveal that just those cells in which differentiation had been induced exhibited neuron filaments and differentiated into neurons.
Primary antibody: mouse monoclonal anti-neurofilament 160
Secondary antibody: HRP-polymer conjugated IgG
Color-developing substrate: Diaminobenzidine (DAB)

The sugar chains having bisect GlcNAc, the sugar chains not having bisect GlcNAc, and the sugar chains of the high mannose type were as follows.

The group of sugar chains having a bisect structure is: Chem. 15, Chem. 22, Chem. 23, Chem. 30, Chem. 36, Chem. 37, Chem. 38, Chem. 44, Chem. 45, Chem. 46, Chem. 48, Chem. 53, Chem. 55, Chem. 56, Chem. 60, Chem. 61, Chem. 64, and Chem. 67 in Table 2.

The group of sugar chains not having a bisect structure is: Chem. 6, Chem. 8 to 10, Chem. 12 to 14, Chem. 17 to 21, Chem. 25 to 29, Chem. 32 to 35, Chem. 40 to 43, Chem. 47, Chem. 50 to 52, Chem. 54, Chem. 57 to 59, Chem. 62, Chem. 63, Chem. 65, and Chem. 66 in Table 2.

The group of sugar chains of the high mannose type is Chem. 5, Chem. 7, Chem. 11, Chem. 16, Chem. 24, Chem. 31, and Chem. 39 in Table 2.

**[Table 2]**

| Structure name | Assumed structure | Structure name | Assumed structure | Structure name | Assumed structure |
|---|---|---|---|---|---|
| Chem.1 | 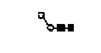 | Chem.25 | 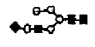 | Chem.49 | A2-amide |
| Chem.2 | 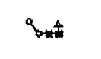 | Chem.26 | 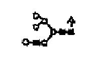 | Chem.50 | 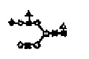 |
| Chem.3 | 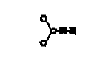 | Chem.27 | 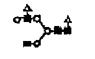 | Chem.51 | 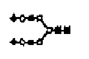 |
| Chem.4 | 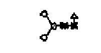 | Chem.28 | 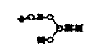 | Chem.52 | 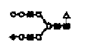 |
| Chem.5 | 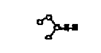 | Chem.29 | 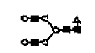 | Chem.53 | 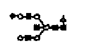 |
| Chem.6 | 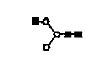 | Chem.30 | 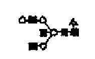 | Chem.54 | 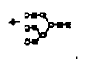 |
| Chem.7 | 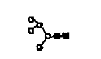 | Chem.31 | 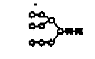 | Chem.55 | 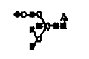 |
| Chem.8 | 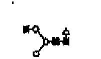 | Chem.32 | 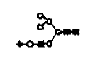 | Chem.56 | 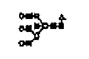 |
| Chem.9 | 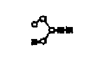 | Chem.33 | 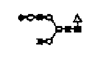 | Chem.57 | 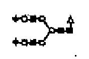 |
| Chem.10 | 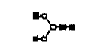 | Chem.34 | 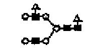 | Chem.58 | 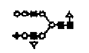 |
| Chem.11 | 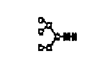 | Chem.35 | 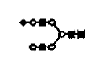 | Chem.59 | 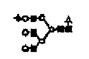 |
| Chem.12 | 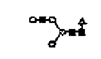 | Chem.36 | 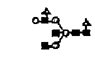 | Chem.50 | 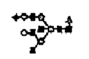 |
| Chem.13 | 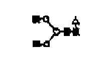 | Chem.37 | 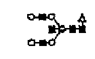 | Chem.61 | 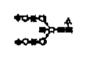 |
| Chem.14 | 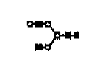 | Chem.38 | 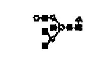 | Chem.62 | 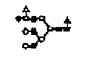 |
| Chem.15 | 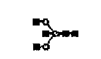 | Chem.39 | 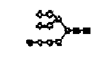 | Chem.63 | 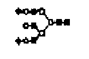 |
| Chem.16 | 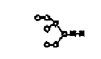 | Chem.40 | 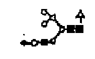 | Chem.64 | 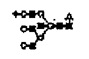 |
| Chem.17 | 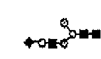 | Chem.41 | 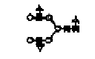 | Chem.65 | 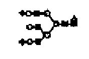 |
| Chem.18 | 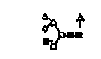 | Chem.42 | 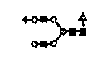 | Chem.66 | 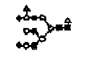 |
| Chem.19 | 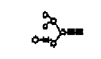 | Chem.43 | 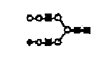 | Chem.67 | 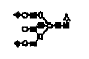 |
| Chem.20 | 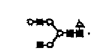 | Chem.44 | 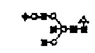 | Chem.68 | 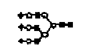 |
| Chem.21 | 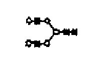 | Chem.45 | 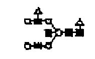 | Chem.69 | 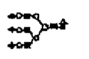 |
| Chem.22 | 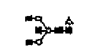 | Chem.46 | 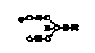 | Chem.70 | 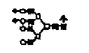 |
| Chem.23 | 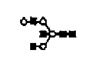 | Chem.47 | 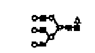 | Chem.71 | 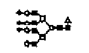 |

In table, ■ donates N-acetyl glucosamine (GlcNAc), Δdonates Fucose (Fuc), ○ (grayish) donates Mannose (Man), Odonates Galactose (Gal), ● donates Glucose (Glc), ◊ donates N-acetyl sialic acid (Neu Ac) and ◊(grayish) donates N-glycolyl sialic acid (Neu Gc).

### Embodiment 3

Differentiation of mouse ES cells was induced with retinoic acid, and glycomixes of undifferentiated and differentiated cells were prepared. The induction of differentiation and the quantitative analysis of the levels of sugar chains expressed were conducted in the same manner as in Embodiment 2. The results of the quantitative analysis of the levels of sugar chains expressed are given in Figs. 3-1 and 3-2 (an enlargement of the y-axis in Fig. 3-1). Based on these results, sub-grouping was conducted into structures thought to have bisect GlcNAc and structures thought to not have it. The results are given in Fig. 3-3. The results indicated that cell differentiation could be quantitatively monitored by this method.

The sugar chains having bisect GlcNAc, the sugar chains not having bisect GlcNAc, and the sugar chains of the high mannose type were as follows.

The group of sugar chains having a bisect structure is: Chem. 29, Chem. 37, Chem. 38, Chem. 58, Chem. 59, Chem. 60, and Chem. 67 in Table 3.

The group of sugar chains not having a bisect structure is: Chem. 4, Chem. 7, Chem. 8, Chem. 11, Chem. 13, Chem. 16, Chem. 18, Chem. 19, Chem. 20, Chem. 26, Chem. 27, Chem. 31, Chem. 32, Chem. 33, Chem. 34, Chem. 35, Chem. 36, Chem. 40, Chem. 41, Chem. 42, Chem. 43, Chem 44, Chem. 45, Chem. 46, Chem. 47, Chem. 52, Chem. 53, Chem. 54, Chem. 55, Chem. 56, Chem. 57, Chem. 62, Chem. 63, Chem. 64, Chem. 65, Chem. 69, Chem. 70, Chem. 71, and Chem. 72 in Table 3.

The group of sugar chains of the high mannose type is: Chem. 12, Chem. 17, Chem. 22, Chem. 30, Chem. 39, Chem. 51, and Chem. 61 in Table 3.

**[Table 3]**

| Structure name | Assumed structure | | Structure name | Assumed structure | | Structure name | Assumed structure |
|---|---|---|---|---|---|---|---|
| Chem.1 | 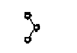 | | Chem.26 | 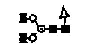 | | Chem.51 | 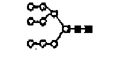 |
| Chem.2 | 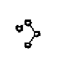 | | Chem.27 | 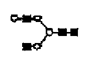 | | Chem.52 | 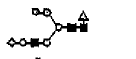 |
| Chem.3 | 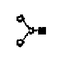 | | Chem.28 | 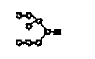 | | Chem.53 | 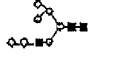 |
| Chem.4 | 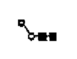 | | Chem.29 | 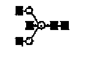 | | Chem.54 | 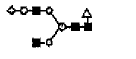 |
| Chem.5 | 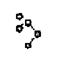 | | Chem.30 | 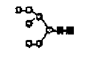 | | Chem.55 | 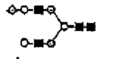 |
| Chem.6 | 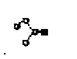 | | Chem.31 | 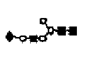 | | Chem.56 | 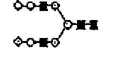 |
| Chem.7 | 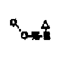 | | Chem.32 | 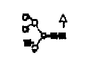 | | Chem.57 | 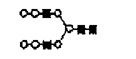 |
| Chem.8 | 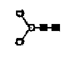 | | Chem.33 | 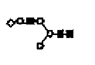 | | Chem.58 | 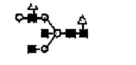 |
| Chem.9 | 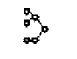 | | Chem.34 | 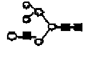 | | Chem.59 | 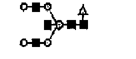 |
| Chem.10 | 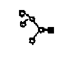 | | Chem.35 | 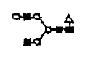 | | Chem.60 | 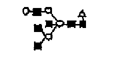 |
| Chem.11 | 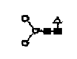 | | Chem.36 | 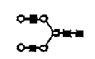 | | Chem.61 | 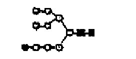 |
| Chem.12 | 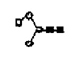 | | Chem.37 | 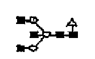 | | Chem.62 | 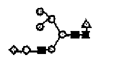 |
| Chem.13 | 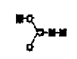 | | Chem.38 | 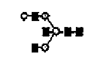 | | Chem.63 | 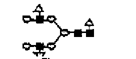 |
| Chem.14 | 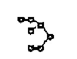 | | Chem.39 | 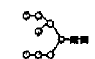 | | Chem.64 | 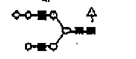 |
| Chem.15 | 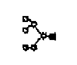 | | Chem.40 | 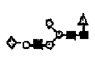 | | Chem.65 | 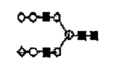 |
| Chem.16 | 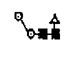 | | Chem.41 | 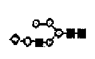 | | Chem.66 | 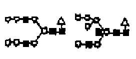 |
| Chem.17 | 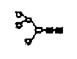 | | Chem.42 | 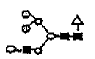 | | Chem.67 | 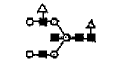 |
| Chem.18 | 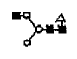 | | Chem.43 | 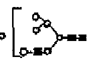 | | Chem.68 | Internal Standard |
| Chem.19 | 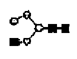 | | Chem.44 | 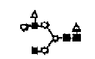 | | Chem.69 | 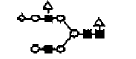 |
| Chem.20 | 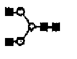 | | Chem.45 | 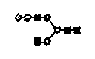 | | Chem.70 | 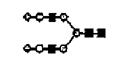 |
| Chem.21 | 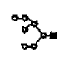 | | Chem.46 | 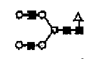 | | Chem.71 | 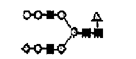 |
| Chem.22 | 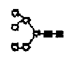 | | Chem.47 | 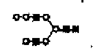 | | Chem.72 | 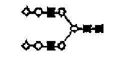 |

In table, ■ donates N-acetyl glucosamine (GlcNAc), Δdonates Fucose (Fuc), ○ (grayish) donates Mannose (Man), Odonates Galactose (Gal), ● donates Glucose (Glc), ◊ donates N-acetyl sialic acid (Neu Ac) and ◊(grayish) donates N-glycolyl sialic acid (Neu Gc).

### Reference Embodiment 4

Differentiation was induced in human ES cells by adding retinoic acid and glycomixes were prepared for undifferentiation prior to the addition of retinoic acid and differentiation following addition. The induction of differentiation and the quantitative analysis of the levels of sugar chains expressed were conducted in the same manner as in Embodiment 2. The results of the quantitative analysis of the levels of sugar chains expressed are given in Figs. 4-1 and 4-2 (an enlargement of the y-axis in Fig. 4-1). Based on these results, sub-grouping was conducted into types having one fucose and types that did not. The results are given in Fig. 4-3. Further, sub-grouping was conducted into types not having sialic acid and sugar chains that did not. The results are given in Fig. 4-4. The results indicated that cell differentiation could be quantitatively monitored by this method.

The sugar chains having one fucose, the sugar chains not having sialic acid, and the sugar chains of the high mannose type were as follows.

The group of sugar chains having one fucose is: Chem. 6, Chem. 11, Chem. 13, Chem. 17, Chem. 19, Chem. 32, Chem. 36, Chem. 37, Chem. 39, Chem. 44, Chem. 47, Chem. 50, Chem. 51, and Chem. 52 in Table 4.

The group of sugar chains not having sialic acid is: Chem. 6, Chem. 7, Chem. 8, Chem. 11, Chem. 13, Chem. 14, Chem. 17, Chem. 19, Chem. 20, Chem. 34, Chem. 35, Chem. 36, Chem. 37, Chem. 38, Chem. 39, Chem. 42, Chem. 44, Chem. 46, Chem. 47, Chem, 49, and Chem. 52 in Table 4.

The group of sugar chains of the high mannose type is: Chem. 12, Chem. 18, Chem. 22, Chem. 26, Chem. 31, Chem. 40, and Chem. 48 in Table 4.

**[Table 4]**

| Structure name | Assumed structure | | Structure name | Assumed structure |
|---|---|---|---|---|
| Chem.1 | 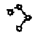 | | Chem.31 | 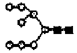 |
| Chem.2 | 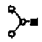 | | Chem.32 | 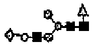 |
| Chem.3 | 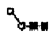 | | Chem.33 | 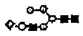 |
| Chem.4 | 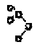 | | Chem.4 | 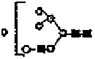 |
| Chem.5 | 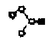 | | Chem.35 | 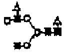 |
| Chem.8 | 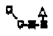 | | Chem.36 | 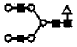 |
| Chem.7 | 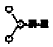 | | Chem.37 | 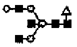 |
| Chem.8 | 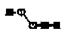 | | Chem.38 | 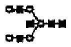 |
| Chem.9 | 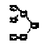 | | Chem.39 | 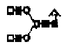 |
| Chem.10 | 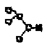 | | Chem.40 | 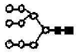 |
| Chem.11 | 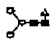 | | Chem.41 | 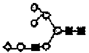 |
| Chem.12 | 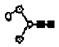 | | Chem.42 | 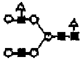 |
| Chem.13 | 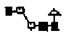 | | Chem.43 | 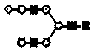 |
| Chem.14 | 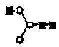 | | Chem.44 | 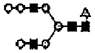 |
| Chem.15 | 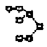 | | Chem.45 | 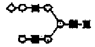 |
| Chem.16 | 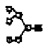 | | Chem.46 | 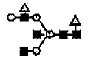 |
| Chem.17 | 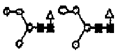 | | Chem.47 | 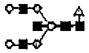 |
| Chem.18 | 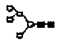 | | Chem.48 | 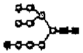 |
| Chem.19 | 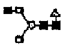 | | Chem.49 | 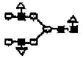 |
| Chem.20 | 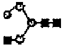 | | Chem.50 | 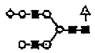 |
| Chem.21 | 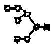 | | Chem.51 | 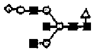 |
| Chem.22 | 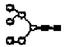 | | Chem.52 | 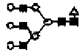 |

In table, ■ donates N-acetyl glucosamine (GlcNAc), Δdonates Fucose (Fuc), ○ (grayish) donates Mannose (Man), Odonates Galactose (Gal), ● donates Glucose (Glc), ◊ donates N-acetyl sialic acid (Neu Ac) and ◊(grayish) donates N-glycolyl sialic acid (Neu Gc).

### [Industrial Applicability]

The present invention is useful in the fields of manufacturing and developing pharmaceuticals and regenerative therapy relating to, for example, the differentiation of stem cells.

## Claims

1. A method for categorizing sugar chains employed to evaluate the level of differentiation of stem cells or precursor cells in which differentiation has been induced **characterized by**:
obtaining a quantitative profile of the N-bond-type sugar chains contained in stem cells or precursor cells cultivated in the absence of a differentiation-inducing agent;
obtaining a quantitative profile of the N-bond-type sugar chains contained in stem cells or precursor cells cultivated in the presence of a differentiation-inducing agent, wherein the N-bond-type sugar chains are those separated and purified by the glycoblotting method; extracting at least a part of the group of sugar chains in which the quantities present in the two quantitative profiles have varied;
categorizing the various sugar chains contained in the group of sugar chains extracted based on type of sugar chain into high mannose type and non-high mannose type and further dividing the non-high mannose type into categories based on (1) the presence or absence of bisects, (2) the number of fucoses (0, 1, or 2 or more), (3) the presence or absence of sialic acid, and (4) the degree of branching (2 or 3 or more); and
determining at least one category of sugar chain from among the five categories as being suited to evaluation of the level of differentiation of stem cells or precursor cells.

2. A method for evaluating the level of differentiation of stem cells or precursor cells in which differentiation has been induced **characterized by**:
obtaining time-course quantitative profiles of N-bond-type sugar chains contained in stem cells or precursor cells that have been cultured in the presence of a differentiation-inducing agent; wherein the N-bond-type sugar chains are those separated and purified by the glycoblotting method; and
determining from the quantitative profiles the time-course change in the present quantity or present ratio of the sugar chains (groups) contained in at least one of the categories of sugar chain; wherein the at least one of the categories of sugar chain is determined by the method in which
a quantitative profile of the N-bond-type sugar chains contained in stem cells or precursor cells cultivated in the absence of a differentiation-inducing agent is obtained; a quantitative profile of the N-bond-type sugar chains contained in stem cells or precursor cells cultivated in the presence of a differentiation-inducing agent is obtained, wherein the N-bond-type sugar chains are those separated and purified by the glycoblotting method;
at least a part of the group of sugar chains in which the quantities present in the two quantitative profiles have varied is extracted;
the various sugar chains contained in the group of sugar chains extracted based on type of sugar chain are categorized into high mannose type and non-high mannose type and the non-high mannose type is further divided into categories based on (1) the presence or absence of bisects, (2) the number of fucoses (0, 1, or 2 or more), (3) the presence or absence of sialic acid, and (4) the degree of branching (2 or 3 or more); and
at least one category of sugar chain is determined from among the five categories as being suited to evaluation of the level of differentiation of stem cells or precursor cells.

3. The method according to claim 2, wherein the differentiation-inducing agent is a cardiac muscle differentiation-inducing agent; and
the time-course change determined is a time-course change in at least one member selected from the group consisting of the quantity or the ratio of the group of sugar chains having one fucose, the quantity or the ratio of the group of sugar chains having two fucoses, and the quantity or the ratio of the group of sugar chains of the high mannose type present in the quantitative profile to evaluate the level of differentiation of stem cells or precursor cells into cardiac muscle.

4. The method according to claim 3, wherein cells in which the quantity or ratio of the group of sugar chains having one fucose that is present increases with culturing are cells in which the level of differentiation into cardiac muscle has increased.

5. The method according to claim 3, wherein cells in which the quantity or ratio of the group of sugar chains having two fucoses that is present decreases by culturing are cells in which the level of differentiation into cardiac muscle has increased.

6. The method according to claim 3, wherein cells in which the quantity or ratio of the group of sugar chains of the high mannose type that is present decreases with culturing are cells in which the level of differentiation into cardiac muscle has increased.

7. The method according to claim 2, wherein the differentiation-inducing agent is an agent inducing differentiation into neurons; and
the time-course change determined is a time-course change in at least one member selected from the group consisting of the quantity or the ratio of the group of sugar chains having a bisect structure, the quantity or the ratio of the group of sugar chains not having a bisect structure, and the quantity or ratio of the group of sugar chains of the high mannose type present in the quantitative profile to evaluate the level of differentiation of stem cells or precursor cells derived from mice into neurons.

8. The method according to claim 7, wherein cells in which the quantity or ratio of the group of sugar chains having a bisect structure that is present increases with culturing are cells in which the level of differentiation into neurons has increased; wherein cells in which the quantity or ratio of the group of sugar chains not having a bisect structure that is present decreases with culturing are cells in which the level of differentiation into neurons has increased.

9. The method according to claim 7, wherein cells in which the quantity or ratio of the group of sugar chains of the high mannose type that is present decreases with culturing are cells in which the level of differentiation into neurons has increased.

10. The method according to claim 2, wherein the differentiation-inducing agent is an agent inducing differentiation into neurons; and
the time-course change determined is a time-course change in at least one member selected from the group consisting of the quantity or the ratio of the group of sugar chains having one fucose, the quantity or the ratio of the group of sugar chains not having sialic acid, and the quantity or ratio of the group of sugar chains of the high mannose type present in the quantitative profile to evaluate the level of differentiation of stem cells or precursor cells derived from humans into neurons.

11. The method according to claim 10, wherein cells in which the quantity or ratio of the group of sugar chains having one fucose that is present increases with culturing are cells in which the level of differentiation into neurons has increased; wherein cells in which the quantity or ratio of the group of sugar chains not having sialic acid that is present increases with culturing are cells in which the level of differentiation into neurons has increased.

12. The method according to claim 10, wherein cells in which the quantity or ratio of the group of sugar chains of the high mannose type that is present decreases with culturing are cells in which the level of differentiation into neurons has increased.

13. The method according to any one of claims 1 to 12, wherein the quantitative profiles are those obtained through the use of MALDI-TOF/MS or LC-ESI/SSI-TOF/MS.

14. The method according to any one of claims 1 to 13, wherein the stem cell is embryonic stem cells, tissue stem cells, adult stem cells, or induced pluripotent stem cells (iPS cells).

15. The method according to any one of claims 1 to 14, wherein the precursor cell is a cell obtained by inducing differentiation in a stem cell and is pluripotent.

## Patentansprüche

1. Verfahren zum Kategorisieren von Zuckerketten eingesetzt zur Beurteilung des Grades der Differenzierung von Stammzellen oder Vorläuferzellen, bei denen Differenzierung induziert wurde, **gekennzeichnet durch**:
Erhalten eines quantitativen Profils von Zuckerketten mit N-Bindung, welche in Stammzellen oder Vorläuferzellen enthalten sind, die in Abwesenheit eines die Differenzierung induzierenden Agens kultiviert wurden;
Erhalten eines quantitativen Profils von Zuckerketten mit N-Bindung, welche in Stammzellen oder Vorläuferzellen enthalten sind, die in Anwesenheit eines die Differenzierung induzierenden Agens kultiviert wurden; wobei die Zuckerketten mit N-Bindung solche sind, die mit Hilfe des Glykoblot-Verfahrens abgetrennt und aufgereinigt werden;
Extrahieren zumindest eines Teils der Gruppe von Zuckerketten, bei denen die in den beiden quantitativen Profilen vorhandenen Mengen variiert haben;
Kategorisieren der verschiedenen Zuckerketten, die in der Gruppe der extrahierten Zuckerketten enthalten waren, auf Grundlage des Typs der Zuckerkette in mannosereich und nicht mannosereich und weitere Einteilung des nicht mannosereichen Typs in Kategorien basierend auf (1) das Vorhandensein oder Fehlen einer Zweiteilung, (2) der Anzahl der Fucose-Einheiten (0, 1 oder 2 oder mehr), (3) das Vorhandensein oder Fehlen von Sialinsäure, und (4) dem Verzweigungsgrad (2 oder 3 oder mehr); und
Bestimmen zumindest einer Kategorie von Zuckerketten aus den fünf Kategorien als geeignet zur Bewertung des Grads der Differenzierung von Stammzellen oder Vorläuferzellen.

2. Verfahren zur Beurteilung des Grades der Differenzierung von Stammzellen oder Vorläuferzellen, bei denen Differenzierung induziert wurde, **gekennzeichnet durch**:
Erhalten von quantitativen Profilen mit Zeitverlauf von Zuckerketten mit N-Bindung, welche in Stammzellen oder Vorläuferzellen enthalten sind, die in Anwesenheit eines die Differenzierung induzierenden Agens kultiviert wurden; wobei die Zuckerketten mit N-Bindungen solche sind, die mit Hilfe des Glykoblot-Verfahrens abgetrennt und aufgereinigt werden; und
Bestimmen der über den Verlauf der Zeit gegebene Änderung der vorliegenden Menge oder des vorliegenden Verhältnisses der Zuckerketten (Gruppen) aus den quantitativen Profilen, die in mindestens einer der Kategorien von Zuckerketten enthalten sind; wobei die mindestens eine der Kategorien von Zuckerketten **durch** das Verfahren bestimmt wird wobei
ein quantitatives Profil der Zuckerketten mit N-Bindung erhalten wird, welche in Stammzellen oder Vorläuferzellen enthalten sind, die in Abwesenheit eines die Differenzierung induzierenden Agens kultiviert wurden;
ein quantitatives Profil der Zuckerketten mit N-Bindung erhalten wird, welche in Stammzellen oder Vorläuferzellen enthalten sind, die in Anwesenheit eines die Differenzierung induzierenden Agens kultiviert wurden, wobei die Zuckerketten mit N-Bindungen solche sind, die mit Hilfe des Glykoblot-Verfahrens abgetrennt und aufgereinigt werden;
zumindest ein Teil der Gruppe von Zuckerketten, bei denen die in den beiden quantitativen Profilen vorhandenen Mengen variiert haben, extrahiert wird;
die verschiedenen Zuckerketten, die in der Gruppe der Zuckerketten enthalten waren und die auf Grundlage des Zuckerkettentyps extrahiert wurden, in mannosereich und nicht mannosereich kategorisiert werden und der nicht mannosereiche Typ weiter in Kategorien eingeteilt wird basierend auf (1) das Vorhandensein oder Fehlen einer Zweiteilung, (2) der Anzahl der Fucose-Einheiten (0, 1 oder 2 oder mehr), (3) das Vorhandensein oder Fehlen von Sialinsäure, und (4) dem Verzweigungsgrad (2 oder 3 oder mehr); und
zumindest eine Kategorie von Zuckerketten aus den fünf Kategorien als geeignet bestimmt wird, zur Bewertung des Grads der Differenzierung von Stammzellen oder Vorläuferzellen.

3. Das Verfahren gemäß Anspruch 2, wobei das die Differenzierung induzierende Agens ein die Differenzierung in Herzmuskelzellen induzierendes Agens ist; und
die festgestellte Änderung über den Zeitverlauf eine Änderung über den Zeitverlauf von mindestens einem Merkmal ist ausgewählt aus der Gruppe bestehend aus der Menge oder dem Verhältnis der Gruppe von Zuckerketten, die eine Fucose-Einheit aufweisen, der Menge oder dem Verhältnis der Gruppe von Zuckerketten, die zwei Fucose-Einheiten aufweisen und der Menge oder dem Verhältnis der Gruppe von Zuckerketten des mannosereichen Typs, die oder das in dem quantitativen Profil enthalten ist, um den Grad der Differenzierung der Stammzellen oder Vorläuferzellen in Herzmuskelzellen zu bewerten.

4. Das Verfahren gemäß Anspruch 3, wobei Zellen, bei denen die Menge oder das Verhältnis der Gruppe von Zuckerketten, die eine Fucose-Einheit enthalten, im Verlauf der Kultivierung steigt, Zellen sind, bei denen der Grad der Differenzierung in Herzmuskelzellen erhöht ist.

5. Das Verfahren gemäß Anspruch 3, wobei Zellen, bei denen die Menge oder das Verhältnis der Gruppe von Zuckerketten, die zwei Fucose-Einheiten enthalten, sich im Verlauf der Kultivierung verringert, Zellen sind, bei denen der Grad der Differenzierung in Herzmuskelzellen erhöht ist.

6. Das Verfahren gemäß Anspruch 3, wobei Zellen, bei denen die Menge oder das Verhältnis der Gruppe von Zuckerketten des mannosereichen Typs sich im Verlauf der Kultivierung verringert, Zellen sind, bei denen der Grad der Differenzierung in Herzmuskelzellen erhöht ist.

7. Das Verfahren gemäß Anspruch 2, wobei das die Differenzierung induzierende Agens ein die Differenzierung in Neuronen induzierendes Agens ist; und die festgestellte Änderung über den Zeitverlauf eine Änderung über den Zeitverlauf von mindestens einem Merkmal ist ausgewählt aus der Gruppe bestehend aus der Menge oder dem Verhältnis der Gruppe von Zuckerketten, die eine Zweiteilung aufweisen, der Menge oder dem Verhältnis der Gruppe von Zuckerketten, die keine Zweiteilung aufweisen und der Menge oder dem Verhältnis der Gruppe von Zuckerketten des mannosereichen Typs, die oder das in dem quantitativen Profil enthalten ist, um den Grad der Differenzierung der aus Mäusen stammenden Stammzellen oder Vorläuferzellen in Neuronen zu bewerten.

8. Das Verfahren gemäß Anspruch 7, wobei Zellen, bei denen die Menge oder das Verhältnis der Gruppe von Zuckerketten, die eine Zweiteilungsstruktur aufweisen, im Verlauf der Kultivierung steigt, Zellen sind, bei denen der Grad der Differenzierung in Neuronen erhöht ist; wobei Zellen, bei denen die Menge oder das Verhältnis der Gruppe von Zuckerketten, die keine Zweiteilungsstruktur aufweisen, sich im Verlauf der Kultivierung verringert, Zellen sind, bei denen der Grad der Differenzierung in Neuronen erhöht ist.

9. Das Verfahren gemäß Anspruch 7, wobei Zellen, bei denen die Menge oder das Verhältnis der Gruppe von Zuckerketten des mannosereichen Typs sich im Verlauf der Kultivierung verringert, Zellen sind, bei denen der Grad der Differenzierung in Neuronen erhöht ist.

10. Das Verfahren gemäß Anspruch 2, wobei das die Differenzierung induzierende Agens ein die Differenzierung in Neuronen induzierendes Agens ist; und die festgestellte Änderung über den Zeitverlauf eine Änderung über den Zeitverlauf von mindestens einem Merkmal ist ausgewählt aus der Gruppe bestehend aus der Menge oder dem Verhältnis der Gruppe von Zuckerketten, die eine Fucose-Einheit aufweisen, der Menge oder dem Verhältnis der Gruppe von Zuckerketten, die keine Sialinsäure aufweisen und der Menge oder dem Verhältnis der Gruppe von Zuckerketten des mannosereichen Typs, die oder das in dem quantitativen Profil enthalten ist, um den Grad der Differenzierung der aus Menschen stammenden Stammzellen oder Vorläuferzellen in Neuronen zu bewerten.

11. Das Verfahren gemäß Anspruch 10, wobei Zellen, bei denen die Menge oder das Verhältnis der Gruppe von Zuckerketten, die eine Fucose-Einheit enthalten, im Verlauf der Kultivierung steigt, Zellen sind, bei denen der Grad der Differenzierung in Neuronen erhöht ist; wobei Zellen, bei denen die Menge oder das Verhältnis der Gruppe von Zuckerketten, die keine Sialinsäure enthalten, im Verlauf der Kultivierung steigt, Zellen sind, bei denen der Grad der Differenzierung in Neuronen erhöht ist.

12. Das Verfahren gemäß Anspruch 10, wobei Zellen, bei denen die Menge oder das Verhältnis der Gruppe von Zuckerketten des mannosereichen Typs sich im Verlauf der Kultivierung verringert, Zellen sind, bei denen der Grad der Differenzierung in Neuronen erhöht ist.

13. Das Verfahren gemäß irgend einem der Ansprüche 1 bis 12, wobei die quantitativen Profile solche sind, die durch die Verwendung von MALDI-TOF/MS oder LC-ESI/SSI-TOF/MS erhalten werden.

14. Das Verfahren gemäß irgend einem der Ansprüche 1 bis 13, wobei die Stammzellen embryonale Stammzellen, Gewebestammzellen, adulte Stammzellen oder induzierte pluripotente Stammzellen (iPS-Zellen) sind.

15. Das Verfahren gemäß irgend einem der Ansprüche 1 bis 14, wobei die Vorläuferzelle eine Zelle ist, die durch Induktion der Differenzierung einer Stammzelle erhalten wird, und pluripotent ist.

## Revendications

1. Procédé de catégorisation de chaînes de sucre utilisé pour évaluer le niveau de différenciation de cellules souches ou de cellules précurseurs dans lesquelles la différenciation a été induite, **caractérisé par** :
l'obtention d'un profil quantitatif des chaînes de sucre de type à liaison N contenues dans les cellules souches ou les cellules précurseurs cultivées en l'absence d'un agent induisant la différenciation ;
l'obtention d'un profil quantitatif des chaînes de sucre de type à liaison N contenues dans les cellules souches ou les cellules précurseurs cultivées en présence d'un agent induisant la différenciation, dans lequel les chaînes de sucre de type à liaison N sont celles séparées et purifiées par la méthode de glycoblotting ;
l'extraction d'au moins une partie du groupe de chaînes de sucre dans lequel les quantités présentes dans les deux profils quantitatifs ont varié ;
la catégorisation des différentes chaînes de sucre contenues dans le groupe de chaînes de sucre extrait sur la base du type de chaîne de sucre en type teneur en mannose élevée et en type teneur en mannose non élevée et en outre la division du type teneur en mannose non élevée en catégories sur la base (1) de la présence ou de l'absence d'intercalaires, (2) du nombre de fucoses (0, 1, ou 2 ou plus), (3) de la présence ou de l'absence d'acide sialique, et (4) du degré de ramification (2 ou 3 ou plus) ; et
la détermination d'au moins une catégorie de chaîne de sucre parmi les cinq catégories comme adaptée à l'évaluation du niveau de différenciation des cellules souches ou des cellules précurseurs.

2. Procédé d'évaluation du niveau de différenciation de cellules souches ou de cellules précurseurs dans lesquelles la différenciation a été induite, **caractérisé par** :
l'obtention d'un profil quantitatif au cours du temps de chaînes de sucre de type à liaison N contenues dans les cellules souches ou les cellules précurseurs qui ont été cultivées en présence d'un agent induisant la différenciation ; dans lequel les chaînes de sucre de type à liaison N sont celles séparées et purifiées par la méthode de glycoblotting ; et
la détermination, à partir des profils quantitatifs, de la modification au cours du temps de la quantité présente ou du taux présent des chaînes de sucre (groupes) contenues dans au moins l'une des catégories de chaînes de sucre ; dans lequel la ou les catégories de chaînes de sucre sont déterminées par un procédé dans lequel :
un profil quantitatif des chaînes de sucre de type à liaison N contenues dans les cellules souches ou les cellules précurseurs cultivées en l'absence d'un agent induisant la différenciation est obtenu ;
un profil quantitatif des chaînes de sucre de type à liaison N contenues dans les cellules souches ou les cellules précurseurs cultivées en présence d'un agent induisant la différenciation est obtenu, dans lequel les chaînes de sucre de type à liaison N sont celles séparées et purifiées par la méthode de glycoblotting ;
au moins une partie du groupe des chaînes de sucre dans lequel les quantités présentes dans les deux profils quantitatifs ont varié est extraite ;
les différentes chaînes de sucre contenues dans le groupe de chaînes de sucre extrait sur la base du type de chaîne de sucre sont catégorisées en type teneur en mannose élevée et en type teneur en mannose non élevée et le type teneur en mannose non élevée est en outre divisé en catégories sur la base (1) de la présence ou de l'absence d'intercalaires, (2) du nombre de fucoses (0, 1, ou 2 ou plus), (3) de la présence ou de l'absence d'acide sialique, et (4) du degré de ramification (2 ou 3 ou plus) ; et
au moins une catégorie de chaînes de sucre est déterminée parmi les cinq catégories comme adaptée à l'évaluation du niveau de différenciation des cellules souches ou des cellules précurseurs.

3. Procédé selon la revendication 2, dans lequel l'agent induisant la différenciation est un agent induisant la différenciation en muscle cardiaque ; et
la modification au cours du temps déterminée est une modification au cours du temps d'au moins un élément sélectionné dans le groupe constitué de la quantité ou du taux du groupe de chaînes de sucre ayant un fucose, de la quantité ou du taux du groupe de chaîne de sucre ayant deux fucoses, et de la quantité ou du taux du groupe de chaînes de sucre du type teneur en mannose élevée présent(e) dans le profil quantitatif pour évaluer le niveau de différenciation de cellules souches ou de cellules précurseurs en muscle cardiaque.

4. Procédé selon la revendication 3, dans lequel les cellules dans lesquelles la quantité ou le taux du groupe de chaînes de sucre ayant un fucose qui est présent(e) augmente avec la culture sont des cellules dans lesquelles le niveau de différenciation en muscle cardiaque a augmenté.

5. Procédé selon la revendication 3, dans lequel les cellules dans lesquelles la quantité ou le taux du groupe de chaînes de sucre ayant deux fucoses qui est présent(e) diminue avec la culture sont des cellules dans lesquelles le niveau de différenciation en muscle cardiaque a augmenté.

6. Procédé selon la revendication 3, dans lequel les cellules dans lesquelles la quantité ou le taux du groupe de chaînes de sucre du type à teneur en mannose élevée qui est présent(e) diminue avec la culture sont des cellules dans lesquelles le niveau de différenciation en muscle cardiaque a augmenté.

7. Procédé selon la revendication 2, dans lequel l'agent induisant la différenciation est un agent induisant la différenciation en neurones ; et
la modification au cours du temps déterminée est une modification au cours du temps d'au moins un élément sélectionné dans le groupe constitué de la quantité ou du taux du groupe de chaînes de sucre ayant une structure à intercalaire, de la quantité ou du taux du groupe de chaînes de sucre n'ayant pas de structure à intercalaire, et de la quantité ou du taux du groupe de chaînes de sucre du type teneur en mannose élevée présent(e) dans le profil quantitatif pour évaluer le niveau de différenciation de cellules souches ou de cellules précurseurs dérivées de souris en neurones.

8. Procédé selon la revendication 7, dans lequel les cellules dans lesquelles la quantité ou le taux du groupe de chaînes de sucre ayant une structure à intercalaire qui est présent(e) augmente avec la culture sont des cellules dans lesquelles le niveau de différenciation en neurones a augmenté ; dans lequel les cellules dans lesquelles la quantité ou le taux du groupe de chaînes de sucre n'ayant pas de structure à intercalaire qui est présent(e) diminue avec la culture sont des cellules dans lesquelles le niveau de différenciation en neurones a augmenté.

9. Procédé selon la revendication 7, dans lequel les cellules dans lesquelles la quantité ou le taux du groupe de chaînes de sucre du type à teneur en mannose élevée qui est présent(e) diminue avec la culture sont des cellules dans lesquelles le niveau de différenciation en neurones a augmenté.

10. Procédé selon la revendication 2, dans lequel l'agent induisant la différenciation est un agent induisant la différenciation en neurones ; et
la modification au cours du temps déterminée est une modification au cours du temps d'au moins un élément sélectionné dans le groupe constitué de la quantité ou du taux du groupe de chaînes de sucre ayant un fucose, de la quantité ou du taux du groupe de chaînes de sucre n'ayant pas d'acide sialique, et de la quantité ou du taux du groupe de chaînes de sucre du type teneur en mannose élevée présent dans le profil quantitatif pour évaluer le niveau de différenciation de cellules souches ou de cellules précurseurs dérivées d'êtres humains en neurones.

11. Procédé selon la revendication 10, dans lequel les cellules dans lesquelles la quantité ou le taux du groupe de chaînes de sucre ayant un fucose qui est présent(e) augmente avec la culture sont des cellules dans lesquelles le niveau de différenciation en neurones a augmenté ; dans lequel les cellules dans lesquelles la quantité ou le taux du groupe de chaînes de sucre n'ayant pas d'acide sialique qui est présent(e) augmente avec la culture sont des cellules dans lesquelles le niveau de différenciation en neurones a augmenté.

12. Procédé selon la revendication 10, dans lequel les cellules dans lesquelles la quantité ou le taux du groupe de chaînes de sucre du type à teneur en mannose élevée qui est présent(e) diminue avec la culture sont des cellules dans lesquelles le niveau de différenciation en neurones a augmenté.

13. Procédé selon l'une quelconque des revendications 1 à 12, dans lequel les profils quantitatifs sont ceux obtenus par l'utilisation de MALDI-TOF/SM ou CL-ESI/SSI-TOF/SM.

14. Procédé selon l'une quelconque des revendications 1 à 13, dans lequel la cellule souche est des cellules souches embryonnaires, des cellules souches tissulaires, des cellules souches adultes, ou des cellules souches pluripotentes induites (cellules iPS).

15. Procédé selon l'une quelconque des revendications 1 à 14, dans lequel la cellule précurseur est une cellule obtenue par induction de la différenciation dans une cellule souche et est pluripotente.
